# EUROPÄISCHE PATENTSCHRIFT

(11) **EP 2 825 098 B1**
(45) Veröffentlichungstag und Bekanntmachung des Hinweises auf die Patenterteilung: **26.09.2018**
(21) Anmeldenummer: 13711610.9
(22) Anmeldetag: 15.03.2013
(51) Int. Cl.: A61B 6/04, A61B 6/00

(54) **DETEKTORANORDNUNG ZUM AUFNEHMEN VON RÖNTGENBILDERN EINES ABZUBILDENDEN OBJEKTS**
DETECTOR ASSEMBLY FOR RECORDING X-RAY IMAGES OF AN OBJECT TO BE IMAGED
DISPOSITIF DÉTECTEUR DESTINÉ À PRENDRE DES IMAGES RADIOGRAPHIQUES D'UN OBJET À REPRÉSENTER

(30) Priorität: 15.03.2012 DE 102012005899
(43) Veröffentlichungstag der Anmeldung: 21.01.2015
(73) Patentinhaber: Fraunhofer Gesellschaft zur Förderung der angewandten Forschung e.V., 80686 München (DE); Charité - Universitätsmedizin, 10117 Berlin (DE)
(72) Erfinder: KEEVE, Erwin, 14469 Potsdam (DE); ENGEL, Sebastian, 48147 Münster (DE); STOPP, Fabian, 10249 Berlin (DE); KÄSEBERG, Marc, 16359 Biesenthal (DE); UHLMANN, Eckart, 25368 Kiebitzreihe (DE)
(74) Vertreter: Pfenning, Meinig & Partner mbB
(86) Internationale Anmeldenummer: PCT/EP2013/055458
(87) Internationale Veröffentlichungsnummer: WO 2013/135888

(56) Entgegenhaltungen:
- WO-A1-03/024333
- DE-A1- 10 332 743
- DE-A1- 19 855 213
- DE-A1-102010 018 627
- DE-B3- 10 352 010
- DE-T2- 69 531 740
- JP-A- 2008 125 611
- JP-A- 2011 101 745
- US-A1- 2001 040 939

## Beschreibung

Die vorliegende Erfindung betrifft eine Detektoranordnung zum Aufnehmen von Röntgenbildern eines abzubildenden Objekts.

Röntgenvorrichtungen dienen zur zerstörungsfreien Prüfung von zu untersuchenden Objekten. In der Medizin werden Röntgenvorrichtungen insbesondere zur Untersuchung von Patienten eingesetzt. Eine von einer Röntgenquelle der Röntgenvorrichtung ausgesandte Röntgenstrahlung durchdringt hierbei das zu untersuchende Objekt teilweise, wobei eine Absorption der Röntgenstrahlung abhängig von einem durchstrahlten Material unterschiedlich stark ausfällt. Ein durch das Objekt hindurchtretender Anteil der Röntgenstrahlung wird von einem Detektor als Projektionsbild des durchleuchteten Objekts detektiert. In dem Projektionsbild werden räumliche Informationen überlagert dargestellt, wohingegen eine dreidimensionale Darstellung beispielsweise des Körperinneren eine exakte Reposition von Knochenbrüchen an Gelenken oder eine genaue Positionierung von Implantaten relativ zu kritischen anatomischen Strukturen ermöglicht.

Zur Erzeugung dreidimensionaler Bilddaten werden mit der Röntgenvorrichtung mehrere zweidimensionale Projektionsaufnahmen eines Objekts aus verschiedenen Raumrichtungen aufgenommen und anschließend über einen Algorithmus das gescannte Volumen rekonstruiert. Computertomographen werden zu diesem Zweck eingesetzt und ermöglichen zwar eine sehr gute Bildqualität, sind allerdings ungeeignet für den intraoperativen Einsatz, da hier der Zugang zum Patienten durch das Gerät versperrt ist. Zusätzlich benötigen derartige Geräte viel Platz, haben eine hohe Strahlenexposition, sind komplex in der Bedienung und verursachen hohe Kosten. Generell existiert bislang keine Möglichkeit, eine Bahn von mehr als 180° ohne bewegte und damit gefährliche Teile nahe bei einem Patienten zu verfahren.

Aus der Patentschrift DE 103 52 010 ist bekannt, zur Erzeugung dreidimensionaler Bilddaten einen Röntgendetektor auf einem verschiebbar gelagerten C-Bogen anzuordnen, der unterhalb einer Patientenlagervorrichtung angeordnet.

Alternativ kann der Röntgendetektor an einem Knickarm befestigt sein, der am Standfuss eines Tisches angeordnet ist, dies wird beispielsweise in der Veröffentlichung mit der Nummer WO03/024333 A1 oder mit der Nummer DE 695 31 740 T2 beschrieben. Der vorliegenden Erfindung liegt daher die Aufgabe zugrunde, eine Detektoranordnung vorzuschlagen, die die genannten Nachteile vermeidet, mit der also mehrere Röntgenaufnahmen aus beliebigen Positionen bei einem möglichst kleinbauenden Detektor gemacht werden können, wobei ein abzubildendes Objekt zwischen den Aufnahmen zugänglich ist.

Diese Aufgabe wird erfindungsgemäß gelöst durch eine Detektoranordnung nach Patentanspruch 1. Vorteilhafte Weiterbildungen sind in den übrigen Ansprüchen beschrieben.

Die vorliegende Anmeldung betrifft eine Detektoranordnung zum Aufnehmen von Röntgenbildern eines auf einer Auflage eines Tisches befindlichen abzubildenden Objekts, mit einem Tisch, der zumindest einen verstellbaren Standfuß zum Bewegen der Auflage aufweist, mit einem Detektor zum Erfassen von Röntgenstrahlung und einer Positioniervorrichtung zum Bewegen des Detektors bezüglich des Objekts, wobei der Detektor in mehrere zu dem Objekt raumfeste Aufnahmepositionen bewegbar ist und die Positioniervorrichtung an dem Standfuß derart befestigt ist, dass die Positioniervorrichtung gemeinsam mit der Auflage bewegt wird und die Positioniervorrichtung einen Knickarm aufweist, der über mindestens eine Rotationsachse und/oder eine Linearachse bewegbar ist.

Große Vorteile ergeben sich daraus, dass die Positioniervorrichtung zum Bewegen des Detektors mit der Auflage regelmäßig mitbewegt wird. Dadurch wird Qualität und Einfachheit der Bildaufnahme eines auf der Auflage liegenden Objektes (vorzugsweise eines menschlichen Patienten) ermöglicht. Außerdem ist der Raumbedarf einer solchen Anordnung gering, da wenig Standfläche im Operationssaal durch die Positioniervorrichtung und den Detektor belegt wird.

Eine Weiterbildung sieht hierbei vor, dass die Positioniervorrichtung zwischen Standfuß und Auflage befestigbar ist. Dies hat den Vorteil, dass die Positioniervorrichtung als zusätzliches Bauteil zwischen einen bestehenden Standfuß und eine bestehende Auflage montiert werden kann. Es ist von daher nicht notwendig, hier eine "komplette Einheit" (bestehend aus Auflage, Positioniervorrichtung, Standfuß und eventuell Röntgenquelle) als kleinste marktfähige Einheit anzusehen; stattdessen kann eine solche Positioniervorrichtung auch zur Nachrüstung bestehender Systeme vorgesehen sein.

Eine Weiterbildung sieht außerdem vor, dass die Auflage zumindest bereichsweise aus einem röntgendurchlässigen Material, beispielsweise einem kohlenstofffaserverstärkten Kunststoff (CFK-Material) besteht. Hierdurch können in der Ruhelage bzw. Ausgangsposition des Detektors Aufnahmen direkt unterhalb der Auflage gemacht werden, um Röntgenaufnahmen einer auf der Auflage befindlichen Person aufzunehmen.

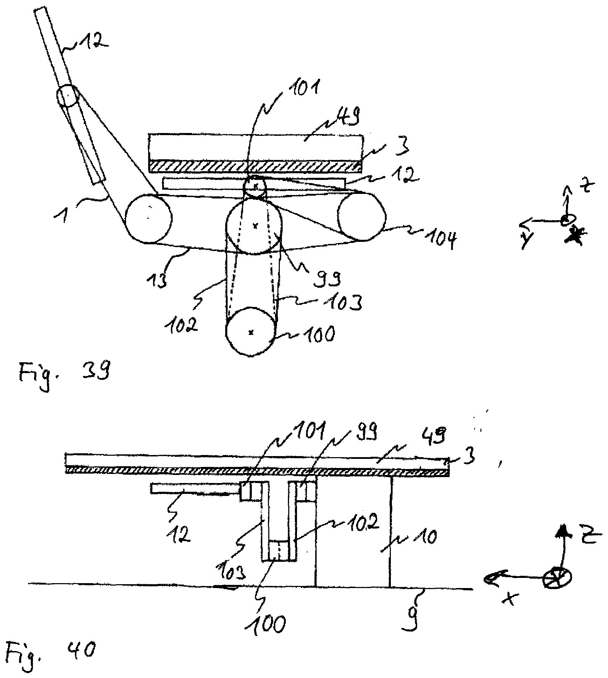

Eine weitere Weiterbildung sieht vor, dass der Standfuß so ausgestaltet ist, dass Auflage und Positioniervorrichtung gemeinsam in der Höhe und/oder in der Neigung verstellbar sind. Hierbei ist die Höhe insbesondere interessant, um Operateure unterschiedlicher Größe einen optimalen Zugriff zum Patienten zu bieten bzw. um sitzende oder stehende Positionen des Operateurs zu begleiten. Vorteilhaft ist auch, dass eine Neigung der Auflage vorgesehen werden kann; dies ist insbesondere zur gezielten Beeinflussung des Gefäßdrucks bei bestimmten medizinischen Indikationen sinnvoll. Die Neigung kann hierbei in einer oder zwei Raumrichtungen verstellbar sein; beispielsweise ist ein Kippen des Patienten zum Operateur hin (Kippen um die Längsachse der Auflage, in den Figuren mit "x-Richtung" bezeichnet, siehe Fig. 39 ff.) möglich. Außerdem ist auch ein Kippen um die y-Achse (siehe Fig. 39 ff.) möglich (siehe oben).

Eine Weiterbildung sieht vor, dass die Auflage auf genau einem Standfuß gehalten ist; dies ist besonders aus Raumgründen sinnvoll, da nur eine zentrale Säule vorgesehen werden muss und die Standfläche um die Auflage / den Patienten herum somit optimal nutzbar ist.

Eine weitere Weiterbildung sieht vor, dass Auflage und Positioniervorrichtung derart bezüglich des Standfußes positioniert sind, dass ein Operateur seine Beine unterhalb der Auflage unterbringen kann. Vorteilhaft ist auch, wenn die Auflage in ihrer Längsrichtung bezüglich des Standfußes linear verschiebbar ist, um die räumliche Ausrichtung und den Platz unterhalb der Auflage besser nutzbar zu machen. Diese Bewegung kann mit der Positioniervorrichtung zusammen und/oder unabhängig von der Positioniervorrichtung erfolgen. Beispielsweise ist es möglich, dass eine Mitführung der Positioniervorrichtung im Gleichklang mit der Auflage erfolgt; zusätzlich kann der Detektor selbst noch einen Linearantrieb zur "Feinjustierung" vorsehen.

Die Positioniervorrichtung weist einen Knickarm in Form eines Roboterarms mit mindestens drei Roboterarmachsen rauf, vorzugsweise können allerdings auch vier, fünf oder sechs Roboterarmachsen zum Positionieren des Detektors vorgesehen sein. Hierbei ist vorteilhaft, wenn drei der Roboterarmachsen parallel zueinander angeordnet sind. Hierdurch werden sehr platzsparende Anordnungen möglich, außerdem ist es leicht möglich, um die Auflage herum eine bogenförmige, elliptische etc. Bahn abzufahren. Auf diese Weise werden gute räumliche Darstellungen erzielt, ohne dass ein sehr platzintensiver "C-Bogen" vorgesehen werden muss.

Vorteilhaft ist außerdem, wenn der Detektor, der vorzugsweise am Ende des Roboterarms (gleichbedeutend mit Knickarm) angebracht ist, zu den Drehfreiheitsgraden durch die Roboterarmachsen noch zusätzlich translatorisch bezüglich der Auflage positionierbar ist.

Es sei betont, dass die Bewegung des Detektors bzw. des Knickarms in sämtlichen Raumrichtungen (d. h. um sämtliche Drehachsen bzw. in sämtlichen linearen Richtungen) motorisch erfolgt. Es ist allerdings auch möglich, hier nichtmotorische Bewegungen vorzusehen und auch vorzusehen, dass die Drehgelenke bzw. Linearführungen selbsthemmend sind, so dass eine Position auch ohne Vorsehen eines Motors gehalten werden kann. Vorteilhaft ist unter anderem, wenn die Längsrichtung der Auflage parallel zu mindestens einer Roboterarmachse, vorzugsweise parallel zu zwei oder drei Roboterarmachsen ist, da dies in Hinblick auf den Platzbedarf des Knickarms/Roboterarms sehr günstig ist.

Vorteilhaft ist auch, wenn die Positioniervorrichtung derart ausgestaltet ist, dass der Detektor von mindestens zwei Seiten der Auflage aus von unterhalb der Auflage nach oberhalb der Auflage bewegbar ist. Dies stellt sicher, dass ein auf einer Seite der Auflage stehender Operateur nicht durch eine auf derselben Seite befindliche Positioniervorrichtung / befindlichen Detektor gestört wird.

Die oben beschriebene Detektoranordnung ist vorzugsweise verwendbar in einer Röntgenvorrichtung, die außerdem noch eine Röntgenquelle aufweist. Hierbei kann es sinnvoll sein, dass die Röntgenquelle an einem weiteren Knickarm/Roboterarm befestigt ist, wobei dieser Knickarm/Roboterarm an der Decke eines Zimmers gehalten ist. Dies ist insbesondere in Hinblick auf den Raumbedarf als auch in Hinblick auf Sterilitätsanforderungen sinnvoll. Die Interaktion der Röntgenquelle mit der Detektoranordnung erfolgt wie weiter unten im Text beschrieben.

Weitere Aspekte bzw. Details der Patentanmeldung, die allerdings nicht alle Bestandteil der Erfindung sind, sind wie folgt: Eine Detektoranordnung zum Aufnehmen von Röntgenbildern eines abzubildenden Objekts umfasst einen Detektor, der zum Erfassen von Röntgenstrahlung dient, und eine Positioniervorrichtung zum Bewegen des Detektors bezüglich des abzubildenden Objekts. Der Detektor ist hierbei auf einer Abbildungsfläche entlang der Positioniervorrichtung in mehrere zu dem Objekt raumfeste Aufnahmepositionen bewegbar. Außerdem weist die Positioniervorrichtung mindestens ein gegenüber der Abbildungsfläche abwinkelbares Seitenelement auf.

Durch das abwinkelbare Seitenelement kann die Detektoranordnung kleinbauender ausgeführt werden, da eine Einstrahlung der Röntgenstrahlung von mehreren Seiten unter beliebigen Winkeln ermöglicht wird, ohne dass die Detektoranordnung eine übermäßig große Fläche einnimmt. Durch die Bewegung des Detektors auf der Abbildungsfläche können unterschiedliche Aufnahmepositionen angefahren werden, die durch die Positioniervorrichtung dennoch räumlich definiert sind und eine eindeutige Zuordnung von Röntgenabbildungen zu Positionen des Detektors erlauben. Somit werden von dem abzubildenden Objekt, in der Medizin typischerweise ein Patient, in definierter Weise Projektionsbilder aufgenommen, die einer weiteren Verarbeitung zugeführt werden können.

Der Detektor ist an einem Knickarm als Positioniervorrichtung angeordnet, wobei der Knickarm über mindestens eine, vorzugsweise mindestens zwei, besonders vorzugsweise mindestens drei Knickachsen und bzw. oder eine Linearachse bewegbar ist und bzw. oder eine Kombination aus der mindestens einen Linearachse und bzw. oder mindestens einer Drehachse beinhaltet. Der Knickarm fungiert somit als Knickmechanik. Der Detektor selbst ist an einem Ende des Knickarms angeordnet.

In einer vorteilhaften Weiterbildung ist der Detektor in das Seitenelement verfahrbar, sodass ohne weitere mechanische Modifikationen der Detektoranordnung auch in dem Seitenelement gelegene Aufnahmepositionen erreicht werden können.

Der Detektor kann auch in mindestens einer auf dem Seitenelement gelegenen Aufnahmeposition komplett außerhalb der Abbildungsfläche liegen. Hierdurch kann die Detektoranordnung besonders platzsparend während Aufnahmen ausfallen, da das Seitenelement so abgewinkelt ist, dass kein Teil des Detektors die Abbildungsfläche schneidet, also eine Breite der Detektoranordnung minimiert wird. Unter der "Abbildungsfläche" soll hierbei eine Fläche verstanden werden, auf der bzw. entlang sich der Detektor, beispielsweise mit seinem Mittelpunkt, bewegen kann. Die Abbildungsfläche soll sich hierbei nicht in das Seitenelement erstrecken, kann jedoch über eine Seitenabbildungsfläche in dem Seitenelement fortgeführt werden. Typischerweise umfasst die Detektoranordnung jedoch zwei Seitenelemente, die sich an einander gegenüberliegenden Teilen der Detektoranordnung befinden. Dies erlaubt es, einen großen Bereich von Einstrahlwinkeln der Röntgenstrahlung abzudecken.

Das mindestens eine Seitenelement ist vorzugsweise in einem Winkel von 15° bis 85°, vorzugsweise 30° bis 70°, besonders vorzugsweise 40° bis 60° gegenüber der Abbildungsfläche in einem mittleren Teil der Detektoranordnung abgewinkelt.

In besonders bevorzugter Weise ist die Positioniervorrichtung U-Förmig, sodass der Detektor in mehrere Positionen rund um eine Mitte es "U" verfahrbar ist. In der Mitte des "U" wird das abzubildende Element platziert, das somit aus verschiedenen Richtungen abgebildet werden kann.

Die Positioniervorrichtung und der Detektor können innerhalb eines Gehäuses angeordnet sein. Hierdurch werden diese Bauteile vor mechanischer Beschädigung geschützt und es besteht, beispielsweise bei Operationen, nicht die Gefahr, dass ein Arzt oder andere im Raum befindliche Personen eines der Geräte unbeabsichtigt beschädigt oder eine ungewollte Kollision während einer automatisierten Bewegung erfolgt. Vorzugsweise umfasst das Gehäuse ein für die Röntgenstrahlung durchlässiges Material oder besteht gänzlich aus einem derartigen Material. Ebenso kann die Positioniervorrichtung aus einem für Röntgenstrahlung durchlässigem Material bestehen oder ein derartiges Material umfassen. Dies erlaubt eine Durchstrahlung des Gehäuses und somit Aufnahmen aus einem größeren Winkelbereich. Unter dem Begriff "Röntgenstrahlung" soll im Rahmen dieser Schrift eine elektromagnetische Strahlung mit einer Wellenlänge zwischen 0,01 nm und 10 nm verstanden werden. In besonders bevorzugter Weise weist das Gehäuse eine geschlossene Oberfläche, also eine Oberfläche ohne Löcher oder Durchbrüche auf, um die Gefahr für den Patienten oder die Gefahr einer mechanischen Beschädigung des Detektors und der Positioniervorrichtung minimal zu halten. Außerdem sind die Positioniervorrichtung und der Detektor typischerweise miteinander verbunden.

In einer vorteilhaften Weiterbildung ist die Detektoranordnung durch eine Halterung abnehmbar an einer Auflage, bei medizinischen Verwendungen typischerweise ein Tisch eines Operationssaals, befestigbar, wobei auf der Auflage das abzubildende Objekt liegt. Diese Befestigung kann sowohl oberhalb als auch unterhalb oder seitlich der Auflage erfolgen. Die Detektoranordnung kann somit an verschiedene Auflagen angebracht und von den Auflagen gelöst werden. Somit ist auch zwischen den Röntgenaufnahmen ein Zugang zu dem abzubildenden Objekt gegeben und die Detektoranordnung kann in verschiedenen Positionen und Lagen variabel angebracht werden.

Besonders vorzugsweise dient das Gehäuse als Auflage, sodass das abzubildende Objekt ohne weitere Bauelemente direkt von der Detektoranordnung gehalten wird.

Der mittlere Teil der Detektoranordnung, der entlang der Abbildungsfläche liegt, ist typischerweise parallel zu einer durch die Auflage verlaufenden Ebene, sofern die Detektoranordnung an der Auflage befestigt wird. Diese durch die Auflage verlaufende Ebene ist in besonders bevorzugter Weise horizontal angeordnet. Der mittlere Teil der Detektoranordnung kann hierzu oberhalb, seitlich zu oder unterhalb der Auflage befestigbar sein.

Das Seitenelement weist vorzugsweise eine Breite auf, die kleiner als eine Breite des mittleren Teils der Detektoranordnung und bzw. oder kleiner als eine Breite der Auflage ist, um eine platzsparende Bauweise zu ermöglichen. In weiteren Ausführungsformen können die Breiten aber auch gleich groß sein, bzw. die Breite des Seitenelements sogar größer als die Breite des mittleren Teils sein. Die Breite der Auflage kann der Breite des mittleren Teils entsprechen.

Das mindestens eine Seitenelement kann abnehmbar anbringbar an die Positioniervorrichtung sein, also sowohl an der Positioniervorrichtung befestigt als auch von dieser gelöst werden. Ferner kann die Detektoranordnung von der Positioniervorrichtung abklappbar sein, beispielsweise durch ein Scharnier, oder aus der Positioniervorrichtung ausziehbar sein. Das Seitenelement kann insbesondere auch unter dem mittleren Teil liegen und zum Aufnehmen von Bildern herausgezogen werden. Im Falle eines abklappbaren Seitenelements wird das Seitenelement typischerweise in seiner Bewegung geführt, beispielsweise durch einen oder mehrere Stäbe. Sollte das Seitenelement nicht benötigt werden, kann es daher platzsparend von der Positioniervorrichtung entfernt werden.

Das Seitenelement ragt vorzugsweise über eine Ebene hinaus, die parallel zu der Auflage durch das abzubildende Objekt verläuft. Hierdurch kann der Detektor auch seitlich zu dem abzubildenden Objekt verfahren werden und Bilder bei einem vollständigen Durchstrahlen des Objekts von der Seite erhalten werden. In besonders bevorzugter Weise kann die Detektoranordnung auch über dem abzubildenden Objekt zugeklappt werden, das Objekt also umschließen. Hierdurch können Aufnahmen des Objekts unter beliebigen Winkeln gemacht werden.

In einer vorteilhaften Weiterbildung umfasst die Positioniervorrichtung mindestens ein Schienensystem, auf dem der Detektor gelagert ist. Dies erlaubt eine mechanisch stabile Lagerung und Bewegung des Detektors. Vorzugsweise werden mehrere Schienensysteme kaskadiert betrieben, also mehrere Schienensysteme zusammengefügt, um eine Bewegung in einer Vielzahl von Freiheitsgraden zu ermöglichen. Die Schienensysteme können, um eine möglichst freie Bewegung des Detektors zu erlauben, zumindest bereichsweise linienförmig, gekrümmt oder freigeformt sein. Alternativ oder zusätzlich kann die Positioniervorrichtung auch einen Linearantrieb und bzw. oder die Knickmechanik umfassen, wobei auch eine Kombination aus dem Linearantrieb mit der Knickmechanik möglich ist.

Es kann vorgesehen sein, dass die Positioniervorrichtung unterhalb, oberhalb und bzw. oder seitlich zu dem abzubildenden Objekt verfahrbar ist. Hierdurch können auch längliche Objekte problemlos abgebildet werden. Hierzu kann die Positioniervorrichtung in einem Standfuß der Auflage oder an einem unterhalb der Auflage geführten Lager angeordnet sein. Alternativ kann die Positioniervorrichtung auch auf einem verschiebbaren Wagen angeordnet sein.

Der Detektor kann durch den Linearantrieb auf der Positioniervorrichtung verfahrbar sein. In einer weiteren Ausführungsform kann der Detektor durch eine Zahnstange und einen Zahntrieb auf einer Lagerführung der Positioniervorrichtung bewegbar sein. Dies erlaubt ein Verfahren in genau einstellbare Positionen. Zudem sind derartige Anordnungen mechanisch stabil und erlauben somit ein zuverlässiges Bewegen des Detektors.

Außerdem kann der Detektor durch einen Motor auf der Positioniervorrichtung verfahrbar sein. Vorzugsweise ist der Detektor in diesem Fall durch eine den Motor steuernde Recheneinheit automatisiert verfahrbar. Dies erlaubt neben einem zuverlässigen Anfahren der Aufnahmepositionen ein selbstständiges Bewegen des Detektors, sodass mehrere Projektionsbilder ohne Eingreifen eines Benutzers erstellt werden können.

Eine Auswertung der von dem Detektor aufgenommenen Projektionsbilder erfolgt vorzugsweise durch eine Auswerteeinheit, die auch Teil einer Recheneinheit sein kann. Die Auswerteeinheit berechnet hierzu eine dreidimensionale Darstellung des abzubildenden Objekts und gibt diese auf einer Ausgabeeinheit, wie beispielsweise einem Bildschirm, für den Benutzer erkennbar aus. Mit der Detektoranordnung können somit zweidimensionale und dreidimensionale Aufnahmen erstellt werden. Zur Erstellung dreidimensionaler Aufnahmen werden hierfür typischerweise mehrere Aufnahmen des Objekts aus unterschiedlichen Winkeln kombiniert.

Vorzugsweise wird der Detektor entlang einer Raumachse und bzw. oder um eine Raumachse rotiert. Die Raumachse kann hierbei eine translatorische Achse, eine rotatorische Achse oder eine freigeformte Achse sein. Die rotatorische Achse kann hierzu senkrecht oder parallel zu einer Oberflächennormalen des Detektors verlaufen. Dies erlaubt eine besonders einfache Transformation von Detektorkoordinaten und somit eine einfache und schnellere Berechnung der Rekonstruktionen. Sofern eine Bewegung um mehrere Raumachsen erfolgt, liegen diese in besonders bevorzugter Weise orthogonal zueinander in einer Ebene.

In einer besonders vorteilhaften Weiterbildung ist der Detektor durch das Bewegen auf der Positioniervorrichtung um eine zentrale Achse rotierbar. Die Oberflächennormale des Detektors weist in diesem Fall stets auf die zentrale Achse, die außerhalb der Positioniervorrichtung liegt, was eine einfache Transformation und somit eine schnelle Verarbeitung von aufgenommenen Daten ermöglicht. Typischerweise verläuft die zentrale Achse durch einen Mittelpunkt des abzubildenden Objekts.

Die Detektoranordnung kann auch mindestens zwei unabhängig voneinander bewegbare Detektoren umfassen, um eine möglichst große Variabilität zu erreichen und möglichst viele Röntgenbilder gleichzeitig zu erhalten.

Der Detektor selbst ist typischerweise ein Flachdetektor, d. h. eine Länge und eine Breite des Detektors sind größer als eine Tiefe des Detektors. Ferner ist der Detektor vorzugsweise ein Festkörperdetektor. Dieser Detektortyp ist gängig für eine Vielzahl von Anwendungen. Eine Längsachse des Detektors und eine Querachse des Detektors liegen bevorzugt in der Abbildungsfläche.

In einer vorteilhaften Weiterbildung ist die Positioniervorrichtung in Bezug auf das abzubildende Objekt raumfest positionierbar. Somit wird die Position des Detektors durch zwei mögliche Bewegungen bestimmt, nämlich die Bewegung des Detektors auf der Positioniervorrichtung und die Bewegung des Detektors gemeinsam mit der Positioniervorrichtung.

Anstelle eines Schienensystems kann auch ein Hubantrieb in Kombination mit einem Rotationsantrieb zum Positionieren der Detektoranordnung bzw. des Detektors verwendet werden. Hierdurch wird eine variable Positionierung des Detektors auf der Positioniervorrichtung ermöglicht.

Eine Auflage zum Aufnehmen eines durch Röntgenbilder abzubildenden Objekts weist in einem Innenraum eine Detektoranordnung mit den beschriebenen Merkmalen auf. Alternativ kann eine Detektoranordnung mit den beschriebenen Merkmalen auch an einer Außenseite der Auflage abnehmbar anbringbar sein. Durch eine feste Verbindung der Detektoranordnung mit der Auflage wird eine mechanische Stabilität erhöht. Demgegenüber weist eine abnehmbare Befestigung eine erhöhte Variabilität auf.

Eine Röntgenvorrichtung umfasst eine Röntgenquelle, eine Auswerteeinheit zum Berechnen zweidimensionaler und bzw. oder dreidimensionaler Rekonstruktionen aus den aufgenommenen Röntgenbildern und eine Detektoranordnung mit den zuvor beschriebenen Merkmalen. Dies erlaubt ein Aufnehmen und Weiterverarbeiten von Projektionsbildern aus unterschiedlichen Winkeln. Die Röntgenquelle ist vorzugsweise an einem Stativarm befestigt und frei in beliebige Positionen bewegbar. Vorzugsweise ist die Röntgenquelle an einem an einer Decke eines Raumes befestigten Roboter bzw. Roboterarm befestigt. Dies ist auch aus Gründen der Sterilität und des Raumbedarfs sinnvoll.

In einer vorteilhaften Weiterbildung umfasst die Röntgenvorrichtung auch die Auflage mit den zuvor beschriebenen Eigenschaften. Somit liegt ein komplettes System vor, mit dem das abzubildende Objekt auch sicher gehalten werden kann.

In besonders bevorzugter Weise ist die Detektoranordnung und bzw. oder die Röntgenquelle jeweils an einem durch mindestens drei Roboterarmachsen bewegbaren Arm eines Roboters angeordnet. Der Arm kann hierbei in mindestens drei Freiheitsgraden, insbesondere in vier Freiheitsgraden, vorzugsweise in fünf Freiheitsgraden, besonders vorzugsweise in sechs Freiheitsgraden bewegbar sein. Die sechs Freiheitsgrade umfassen hierbei drei translatorische und drei rotatorische Freiheitsgrade. Durch die von dem Roboter oder den Robotern durchgeführte Bewegung können die Röntgenquelle und der Röntgendetektor beliebig zueinander positioniert werden, sodass unter einer Vielzahl von frei wählbaren Winkeln Projektionsbilder des abzubildenden Objekts erhalten werden.

Ein Verfahren zum Aufnehmen von Röntgenbildern mit einer Röntgenvorrichtung, die die Röntgenquelle mit den zuvor beschriebenen Merkmalen und die Detektoranordnung mit den zuvor beschriebenen Merkmalen umfasst, weist mehrere Schritte auf. In einem Schritt wird die Röntgenquelle zum Aufnehmen von einzelnen Röntgenbildern des abzubildenden Objekts in mehrere Positionen bewegt. Die Detektoranordnung und bzw. oder der Detektor werden zum Aufnehmen der einzelnen Röntgenbilder bewegt. Durch eine Auswerteeinheit werden schließlich eine Mehrzahl von von dem Detektor aufgenommenen Projektionsbildern als Röntgenbilder aufgenommen. Dies dient zur Rekonstruktion eines dreidimensionalen Modells des abzubildenden Objekts.

Dies ermöglicht es, Aufnahmen des abzubildenden Objekts aus einer Vielzahl von Perspektiven zu erhalten und diese zu einer dreidimensionalen Rekonstruktion weiterzuverarbeiten. Durch das Bewegen der Röntgenquelle und der Detektoranordnung bzw. des Detektors unabhängig voneinander können besagte Elemente in beliebige Positionen zueinander verfahren werden.

Vorzugsweise wird in jeder der Aufnahmepositionen jeweils ein Projektionsbild aufgenommen. Dies erlaubt ein eindeutiges Zuordnen der Projektionsbilder zu den Aufnahmepositionen. Die Detektoranordnung und bzw. oder der Detektor werden zum Aufnehmen vorzugsweise in eine der Röntgenquelle gegenüberliegende Position verfahren, wobei gegenüberliegend nicht zwangsläufig ein orthogonales Auftreffen der Röntgenstrahlen auf den Detektor impliziert. Die Röntgenstrahlung kann unter einem beliebigen Winkel auf den Detektor auftreffen, was das Aufnehmen der Projektionsbilder deutlich vereinfacht.

Um Zeit zu sparen, beispielsweise während einer Operation, können die Röntgenquelle und der Röntgendetektor bzw. der Detektor gleichzeitig bewegt werden.

In bevorzugter Weise werden mehrere abzubildende Gebiete des Objekts (welche auch als Zielgebiet bezeichnet werden) durch zum Teil gleiche Projektionsbilder rekonstruiert, um eine Strahlenbelastung des Objekts durch übermäßig viele Aufnahmen gering zu halten.

Außerdem kann vorgesehen sein, dass zum Aufnehmen der Projektionsbilder die Röntgenquelle eine Bahn, vorzugsweise eine kreisbogenförmige Bahn mit elliptischer Auslenkung von mindestens 90° oberhalb des abzubildenden Objekts durchfährt, um auf einem vordefinierten Weg eine Vielzahl von Projektionsbildern erhalten zu können.

Zum Abbilden und Rekonstruieren eines größeren abzubildenden Zielgebiets, das mit einer einzigen Projektionsbildern nicht erfasst werden kann, und bzw. oder einer Mehrzahl von einzelnen, möglicherweise voneinander beabstandeten Zielgebieten wird typischerweise eine Mehrzahl der Projektionsbilder zu einer zusammenhängenden Projektion bzw. einem einzelnen Projektionsbild des abzubildenden Zielgebiets oder der jeweiligen abzubildenden Zielgebiete zusammengesetzt. Sofern einzelne der Projektionsbilder mehrere der Zielgebiete abbilden, können diese Projektionsbilder mehrfach verwendet werden, um eine vorzugsweise drei-dimensionale Rekonstruktion des jeweils gewünschten Zielgebiets zu erhalten. Das abzubildende Zielgebiet kann somit mit einer reduzierten Anzahl von Projektionsbildern und einer geringeren Strahlenbelastung abgebildet werden.

Die vorliegende Erfindung ist auf die bislang beschriebenen Ausführungsformen nicht begrenzt. Auch die folgenden Ausführungsformen stellen jeweils für sich genommen Erfindungen dar. Diese Ausführungsformen können auch die bereits offenbarten Merkmale der zuvor beschriebenen Weiterbildungen aufweisen. Insbesondere kann eine Röntgenvorrichtung eine Detektoranordnung entsprechend einer der nachfolgenden Ausführungsformen umfassen und ein Verfahren mit dieser Röntgenvorrichtung durchgeführt werden. Das Verfahren kann natürlich auch mit der Detektoranordnung nach einer der nachfolgenden Ausführungsformen durchgeführt werden.

Eine Auflage für ein abzubildendes Objekt umfasst, vorzugsweise in einem Innenraum, eine Detektoranordnung mit einer Positioniervorrichtung und einem Detektor für Röntgenstrahlung, wobei der Detektor auf der Positioniervorrichtung in der Auflage in mindestens zwei Raumachsen bewegbar ist. Hierdurch kann der Detektor einfach an verschiedene Punkte bewegt werden, ohne dass das abzubildende Objekt selbst bewegt werden muss.

Die beiden Raumachsen liegen vorzugsweise in einer horizontalen Ebene, was die Bewegung vereinfacht. Die Positioniervorrichtung kann ein Schienensystem umfassen, das auch kaskadiert sein kann. Hierdurch wird eine mechanisch stabile und zuverlässige Führung des Detektors erreicht. Das Schienensystem kann hierbei die bereits zuvor beschriebenen Eigenschaften aufweisen.

Außerdem kann der Detektor durch einen Linearantrieb, insbesondere einen Motor, mit den zuvor beschriebenen Eigenschaften verfahren werden. Dies erlaubt ein automatisiertes Verfahren des Detektors und ein definiertes Ansteuern ausgesuchter Positionen.

Die Detektoranordnung kann auch unter der Auflage als separates Bauteil angeordnet sein. In bevorzugter Weise ist die Positioniervorrichtung in diesem Fall in einem Gehäuse angeordnet, während der Detektor in mindestens zwei Raumachsen, die bevorzugt in einer horizontalen Ebene liegen, verfahrbar ist. Das Gehäuse kann die zuvor bereits beschriebenen Merkmale aufweisen, also vorzugsweise aus einem für Röntgenstrahlung durchlässigen Material bestehen oder ein derartiges Material umfassen. Die Detektoranordnung ist somit abnehmbar anbringbar an verschiedene Auflagen und kann einfach bewegt oder ausgetauscht werden.

Vorzugsweise ist der Detektor in einem unterhalb der Auflage beweglichen Gehäuse wie einem Standfuß angeordnet, das in mindestens eine, bevorzugt zwei Raumrichtungen verfahren werden kann. Das Gehäuse kann hierzu über ein Linearlager mit der Auflage verbunden sein, und besonders bevorzugt keinen Kontakt mit einem Boden, auf dem die Auflage steht, aufweisen.

Die Positioniervorrichtung kann mindestens ein Seitenelement aufweisen, in das der Detektor vorzugsweise verfahren werden kann. Dieses Seitenelement ist typischerweise abnehmbar anbringbar oder abklappbar von einem Mittelteil angeordnet. Das Seitenelement kann auch in einer Ebene mit einem mittleren Teil der Positioniervorrichtung liegen. Eine Abbildungsfläche verläuft entlang des mittleren Teils und kann sich im Fall von dem an das mittlere Teil angebrachten Seitenelements auch als Seitenabbildungsfläche entlang des Seitenelements fortsetzen.

Der Detektor ist vorzugsweise ein Flachdetektor. In besonders bevorzugter Weise ist der Detektor ein Festkörperdetektor. Dies sind gängige Detektorformen, die platzsparend verbaut werden können.

Ausführungsbeispiele von Detektoranordnungen sind in den Zeichnungen dargestellt und werden nachfolgend anhand der Figuren erläutert.

Es zeigen:
- Fig. 1: eine seitliche Ansicht einer Röntgenvorrichtung, die eine Röntgenquelle, eine Detektoranordnung und eine Auflage umfasst,
- Fig. 2: eine um 90° zu der in Fig. 1 dargestellten Ansicht gedrehte Seitenansicht eines weiteren Ausführungsbeispiels der Röntgenvorrichtung,
- Fig. 3: eine Fig. 2 entsprechende Ansicht der Detektoranordnung mit abklappbaren Seitenelementen,
- Fig. 4: die in Fig. 3 gezeigte Detektoranordnung mit abgeklappten Seitenelementen,
- Fig. 5: eine Fig. 3 entsprechende Ansicht einer weiteren Ausführungsform der Detektoranordnung mit dem in verschiedene Aufnahmepositionen verfahrenen Detektor,
- Fig. 6: eine weitere Ausführungsform der in Fig. 5 gezeigten Detektoranordnung mit gekrümmten Seitenelementen,
- Fig. 7: eine Fig. 3 entsprechende Ansicht eines weiteren Ausführungsbeispiels der Detektoranordnung mit einem Schienensystem und zwei Detektoren,
- Fig. 8: eine Fig. 3 entsprechende Ansicht eines weiteren Ausführungsbeispiels der Detektoranordnung mit abgenommenem Seitenelement,
- Fig. 9: eine Fig. 3 entsprechende seitliche Ansicht eines Ausführungsbeispiels der Detektoranordnung mit einer Führung der Seitenelemente,
- Fig. 10: eine Fig. 5 entsprechende Ansicht einer Ausführungsform der Röntgenvorrichtung, bei der der Detektor und die Röntgenquelle in mehrere Aufnahmepositionen bewegbar sind,
- Fig. 11: eine Fig. 10 entsprechende Ansicht eines Ausführungsbeispiels der Röntgenvorrichtung mit mehreren Positionen des Detektors und der Röntgenquelle,
- Fig. 12: eine Draufsicht auf ein geradliniges Schienensystem der Detektoranordnung,
- Fig. 13: eine Fig. 12 entsprechende Ansicht eines Schienensystems mit gekrümmten Schienen,
- Fig. 14: eine Fig. 12 entsprechende Ansicht eines kaskadierten Schienensystems,
- Fig. 15: eine Draufsicht einer Detektoranordnung, die an der Auflage befestigt ist, wobei der Detektor oberhalb der Auflage beweglich ist,
- Fig. 16: eine seitliche Ansicht der in Fig. 15 gezeigten Detektoranordnung,
- Fig. 17: eine seitliche Ansicht einer weiteren Ausführungsform der Röntgeneinrichtung mit beweglicher Röntgenquelle und beweglicher Detektoranordnung,
- Fig. 18: eine seitliche Ansicht einer weiteren Ausführungsform der an der Auflage befestigten Detektoranordnung, bei der der Detektor unterhalb der Auflage verfahrbar ist,
- Fig. 19: eine Fig. 18 entsprechende Darstellung einer weiteren Ausführungsform der an der Auflage verschiebbar befestigten Detektoranordnung,
- Fig. 20: eine Fig. 3 entsprechende Darstellung einer Ausführungsform der Detektoranordnung mit Führung durch einen Zahntrieb,
- Fig. 21: eine Fig. 20 entsprechende Ansicht einer Ausführungsform der Detektoranordnung mit einem Schlitten,
- Fig. 22: eine Fig. 21 entsprechende Darstellung einer Ausführungsform der Detektoranordnung aus Fig. 21 mit einem Linearantrieb,
- Fig. 23: eine seitliche Ansicht einer Ausführungsform der Röntgenvorrichtung, bei der die Detektoranordnung an einem bewegbaren Wagen rotierbar angebracht ist,
- Fig. 24: eine Fig. 23 entsprechende Ansicht einer Ausführungsform der Röntgenvorrichtung, bei der die Detektoranordnung oberhalb der Auflage angeordnet ist,
- Fig. 25: eine seitliche Ansicht einer weiteren Ausführungsform der Röntgenvorrichtung, bei der die Röntgenquelle und die Detektoranordnung jeweils an Roboterarmen befestigt sind und frei positionierbar sind,
- Fig. 26: eine Draufsicht auf eine Aufnahmebahn der Röntgenquelle zum Abfahren mehrerer Aufnahmepositionen,
- Fig. 27: eine seitliche Ansicht der in Fig. 26 gezeigten Aufnahmebahn,
- Fig. 28: eine seitliche Ansicht der Auflage mit integrierter Detektoranordnung,
- Fig. 29: eine Draufsicht auf die in Fig. 28 gezeigte Auflage,
- Fig. 30: eine seitliche Ansicht der Auflage mit unterhalb der Auflage befestigter Detektoranordnung,
- Fig. 31: eine seitliche Ansicht der der Auflage, bei der die Detektoranordnung in einem unterhalb der Auflage befindlichen Tischfuß angeordnet ist,
- Fig. 32: eine seitliche Ansicht einer Ausführungsform der Auflage, bei der die Detektoranordnung unmittelbar unterhalb der Auflage angeordnet ist,
- Fig. 33: eine seitliche Ansicht einer weiteren Ausführungsform der Röntgenvorrichtung, bei der die Detektoranordnung über ein Lager mit der Auflage verbunden ist,
- Fig. 34: eine Fig. 33 entsprechende Ansicht einer weiteren Ausführungsform der Röntgenvorrichtung, bei der die Detektoranordnung in einem beweglichen Gehäuse unterhalb der Auflage angeordnet ist,
- Fig. 35: eine Draufsicht auf den drehbar auf dem Schienensystem gelagerten Detektor,
- Fig. 36: eine Seitenansicht einer Ausführungsform der Detektoranordnung mit an einen mittleren Teil mit anklappbaren Seitenelementen,
- Fig. 37: eine Fig. 36 entsprechende Darstellung, bei der die Positioniervorrichtung ein Schienensystem umfasst,
- Fig. 38: eine seitliche Ansicht einer Ausführungsform der Detektoranordnung, bei der der Detektor durch einen Hubantrieb und einen Rotationsantrieb positionierbar ist,
- Fig. 39: eine seitliche Ansicht einer Ausführungsform der Detektoranordnung, bei der der Detektor über einen Knickarm positionierbar ist,
- Fig. 40: eine um 90° gedrehte Ansicht der in Fig. 39 dargestellten Ausführungsform sowie
- Fign. 41-45: weitere Ansichten der Ausführungsform nach den Figuren 39 und 40.

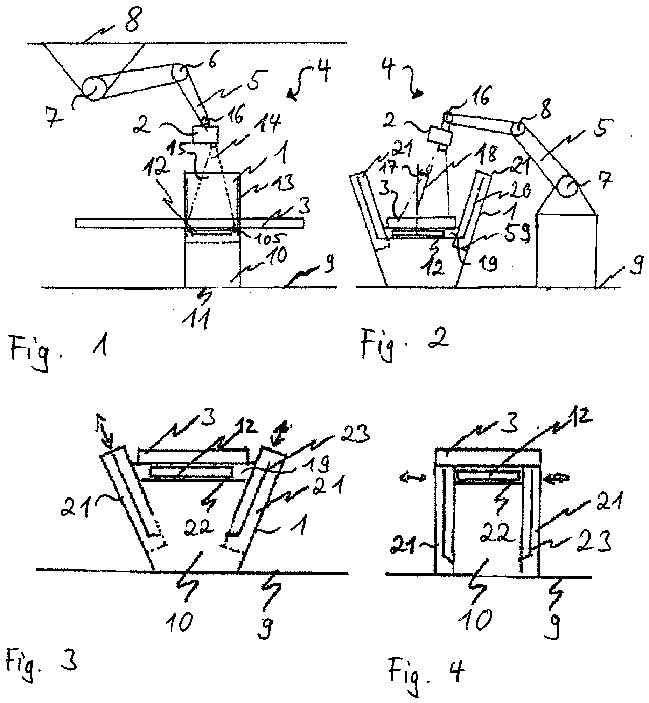

Fig. 1 zeigt eine Detektoranordnung 1, die zusammen mit einer Röntgenquelle 2 und einer Auflage 3 eine Röntgenvorrichtung 4 bildet. Die Röntgenquelle 2 ist an einem Ende eines automatisch bewegbaren Roboterarms 5 befestigt, der über ein erstes Gelenk 6 und ein zweites Gelenk 7 in vier Raumrichtungen bewegbar ist. In weiteren Ausführungsformen kann der Roboterarm 5 auch in sechs Raumrichtungen bewegbar sein, wobei die sechs Raumrichtungen drei translatorische und drei rotatorische Raumrichtungen umfassen. Der Roboterarm 5 ist mit einem der Röntgenquelle 2 entgegengesetzten Ende an einer Decke 8 eines Operationssaals befestigt. Die Röntgenquelle 2 ist über ein drittes Gelenk 16 ebenfalls in mehrere Richtungen und eingerichtet, Röntgenstrahlung verschiedener Wellenlängen und Intensitäten auszusenden. Der Roboterarm 5 wird über eine Recheneinheit in seiner Bewegung gesteuert, kann aber auch manuell als Stativarm positionierbar sein.

Auf einem Boden 9 des Operationssaals ist ein Standfuß 10 über eine Befestigung 11, beispielsweise eine Verschraubung, angebracht. Auf dem Standfuß 10 ist parallel zu dem Boden 9 horizontal die Auflage 3 gelagert, die in dem in Fig. 1 dargestellten Ausführungsbeispiel ein OP-Tisch ist, auf dem ein Patient als durch die Röntgenvorrichtung 4 abzubildendes Objekt aufliegen kann.

Unterhalb der Auflage 3 ist die Detektoranordnung 1 durch eine Klemmhalterung 105 abnehmbar an der Auflage 3 befestigt. Statt Klemmen kann die Detektoranordnung 1 auch an die Auflage 3 geschraubt oder eingeklinkt sein. Alternativ kann die Detektoranordnung 1 auch oberhalb der Auflage 3 oder seitlich zu der Auflage 3 an dieser befestigt sein. Die Detektoranordnung 1 umfasst einen Detektor 12, im dargestellten Ausführungsbeispiel einen Flachdetektor, der parallel zu der Auflage 3 positioniert ist. Außerdem umfasst die Detektoranordnung 1 eine Positioniervorrichtung 13, mit der der Detektor 12 verbunden ist und auf der der Detektor 12 bewegt werden kann. Die Bewegung des Detektors 12 auf der Positioniervorrichtung 13 kann in diesem wie auch in den folgenden Ausführungsbeispielen manuell oder durch einen Motor erfolgen. Die Positioniervorrichtung 13 weist in dem in Fig. 1 dargestellten Ausführungsbeispiel ein Schienensystem auf.

Ein von der Röntgenquelle 2 auf den der Röntgenquelle 2 gegenüberliegend angeordneten Detektor 12 ausgehender Röntgenkegelstrahl 14 trifft auf den Detektor 12, der als Festkörperdetektor ausgebildet ist. Sofern ein abzubildendes Objekt innerhalb des Röntgenkegelstrahls 14 auf der Auflage 3 liegt, wird dieses von der Röntgenstrahlung durchstrahlt. Der Röntgenkegelstrahl 14 kann durch eine bewegliche Blende vor der Röntgenquelle 2 in seinen Abmessungen variiert werden. Die Röntgenstrahlung wird unterschiedlich stark in Abhängigkeit von räumlich in dem Objekt unterschiedlich verteilten Materialien absorbiert, sodass ein von die Röntgenstrahlung mit räumlich unterschiedlicher Intensität auf dem Detektor 12 auftrifft. Hierdurch wird auf dem Detektor 12 ein Projektionsbild erzeugt, das von dem Detektor 12 an eine Auswerteeinheit zur weiteren Bearbeitung weitergeleitet wird und von der Auswerteeinheit schließlich auf einer Ausgabeeinheit dargestellt wird.

Die Detektoranordnung 1 ist in einem Gehäuse 15 aus Kunststoff angeordnet. Das Gehäuse 15 weist eine geschlossene Oberfläche ohne Löcher auf und umschließt die Detektoranordnung 1 vollständig. Das Gehäuse 15 ist durchlässig für die von der Röntgenquelle 2 ausgesandte Röntgenstrahlung, absorbiert die Röntgenstrahlung also nicht oder nur geringfügig. Generell ist das aufgenommene Projektionsbild umso größer, je näher die Röntgenquelle 2 an den Detektor 12 gebracht wird. Je weiter ein Ursprung des Röntgenkegelstrahls 14 vom abzubildenden Zielgebiet entfernt ist, desto größer ist, bei einer gleichzeitigen Verkleinerung eines Röntgenstrahlkegelwinkels, das aufgenommene Zielgebiet. Durch eine freie Positionierung der Röntgenquelle 2 und des Detektors 12 können größere abzubildende Gebiete, worunter insbesondere Gebiete verstanden werden sollen, deren Abmessungen in zumindest einer Richtung größer als ein Kegelstrahldurchmesser sind, mittels einer Superposition unterschiedlicher auf verschiedenen Projektionsaufnahmen enthaltenen Zielgebiete rekonstruiert werden. Hierbei wird ein Teil der Projektionsbilder für zwei oder mehr Zielgebiete verwendet, wodurch eine relative Dosis für das abzubildende Objekt verringert wird.

In weiteren Ausführungsbeispielen können die Auflage 3 und die Detektoranordnung 1 auch einteilig ausgeführt sein, d. h. dass die Auflage 3 zumindest in einem Teil ihres Innenraums die Detektoranordnung 1 umfasst.

Fig. 2 zeigt ein weiteres Ausführungsbeispiel der Röntgenvorrichtung 4 in einer seitlichen Ansicht, die allerdings um 90° gegenüber der in Fig. 1 dargestellten Ansicht gedreht wurde. Wiederkehrende Merkmale sind in dieser Figur wie auch in den nachfolgenden Figuren mit identischen Bezugszeichen versehen. Die Röntgenvorrichtung 4 umfasst wiederum die Röntgenquelle 2, die Detektoranordnung 1 und die Auflage 3. Die Röntgenquelle 2 ist an dem Roboterarm 5 angeordnet, der nun allerdings auf dem Boden 9 über eine Klemmverbindung befestigt ist. Wie in dem in Fig. 1 gezeigten Ausführungsbeispiel weist der Roboterarm 5 drei Gelenke 7, 8 und 16 auf. Der Röntgenkegelstrahl 14, der von der Röntgenquelle 2 aus auf den Detektor 12 trifft ist nun gegenüber einer Oberflächennormalen 17 des Detektors 12 um eine Winkelabweichung 18 verkippt und ein Röntgenzentralstrahl trifft nicht orthogonal auf den Detektor 12 auf. Die Winkelabweichung 18 beschreibt eine Abweichung eines Röntgenzentralstrahls des Röntgenkegelstrahls 14 von der Oberflächennormalen 17. Der Röntgenzentralstrahl ist hierbei ein mittig in dem Röntgenkegelstrahl 14 befindlicher Strahl. Da der Röntgenkegelstrahl 14 vor dem Auftreffen auf den Detektor 12 die Auflage 3 durchlaufen muss, ist diese ebenfalls aus einem für Röntgenstrahlung durchlässigen Material gefertigt.

Die Detektoranordnung 1 weist einen mittleren Teil 19 sowie zwei von dem mittleren Teil 19 abwinkelbare Seitenelemente 21 auf. Die Seitenelemente 21 sind gegenüber dem Mittelteil abklappbar und absenkbar. Der mittlere Teil 19 ist parallel zu einer durch die Auflage 3 verlaufenden Ebene und horizontal. Der mittlere Teil 19 ist in Fig. 2 zwar unterhalb der der Auflage 3 angebracht, kann jedoch auch oberhalb oder seitlich zu ihr liegen. Die Seitenelemente 21 liegen einander gegenüber an dem mittleren Teil 19 und sind mit diesem verbunden, sodass der Detektor 12 auch in die Seitenelemente 21 verfahrbar ist. Die Seitenelemente 21 sind in dem in Fig. 3 dargestellten Ausführungsbeispiel an einem unteren Ende 59 offen, um den Detektor 12 für Wartungsarbeiten schnell aus der Detektoranordnung 1 entfernen und in diese wieder einsetzen zu können. In weiteren Ausführungsbeispielen können die Seitenelemente an dem unteren Ende 59 natürlich auch geschlossen sein.

In Fig. 3 ist die Auflage 3 mit daran befestigter Detektoranordnung 1 in einer Fig. 2 entsprechenden Seitenansicht ohne Roboterarm 5 und Röntgenquelle 2 dargestellt. Die Auflage 3 ruht wiederum auf dem mit dem Boden 9 verbundenen Standfuß 10, im Gegensatz zu der Darstellung der Fig. 2 sind die Seitenelemente 21 nun jedoch durch eine durch Pfeile angedeutete Bewegung abgeklappt, indem sie neben den mittleren Teil 19 der Detektoranordnung 1 gefahren wurden. Durch die gegenüber Fig. 2 vergrößerte Darstellung ist das Schienensystem 23 in den Seitenelementen 21 erkennbar, das mittig zwischen zwei die Seitenelemente 21 begrenzenden Oberflächen angeordnet ist. Der Detektor 12 ist über ein Bewegungslager 22 auf dem Schienensystem 23 geführt. Die Seitenelemente 21 können beispielsweise für eine Aufnahme angeklappt werden und nachfolgend von der Auflage 3 nach unten weggeklappt werden. Hierdurch ist freier Zugang zu dem abzubildenden Objekt gegeben. Nach Ausrichten oder anderweitigem Behandeln des abzubildenden Objekts können die Seitenelemente 21 für weitere Aufnahmen wieder angeklappt werden.

Fig. 4 zeigt die in Fig. 3 dargestellte Detektoranordnung 1 mit vollständig abgeklappten Seitenelementen 21. Die Seitenelemente 21 sind vollständig unterhalb der Auflage 3, sodass ein freier Zugang zu einem auf der Auflage 3 befindlichen Objekt gegeben ist. Durch eine durch horizontale Pfeile angedeutete Bewegung werden die Seitenelemente 21 zum Anklappen zunächst aus der in Fig. 4 gezeigten Position entfernt und anschließend neben der Auflage 3 vorbeigeführt. Ein Verstauen unterhalb der Auflage 3 kann hierbei generell durch eine oder mehrere lineare und bzw. oder eine oder mehrere drehende Bewegungen erfolgen.

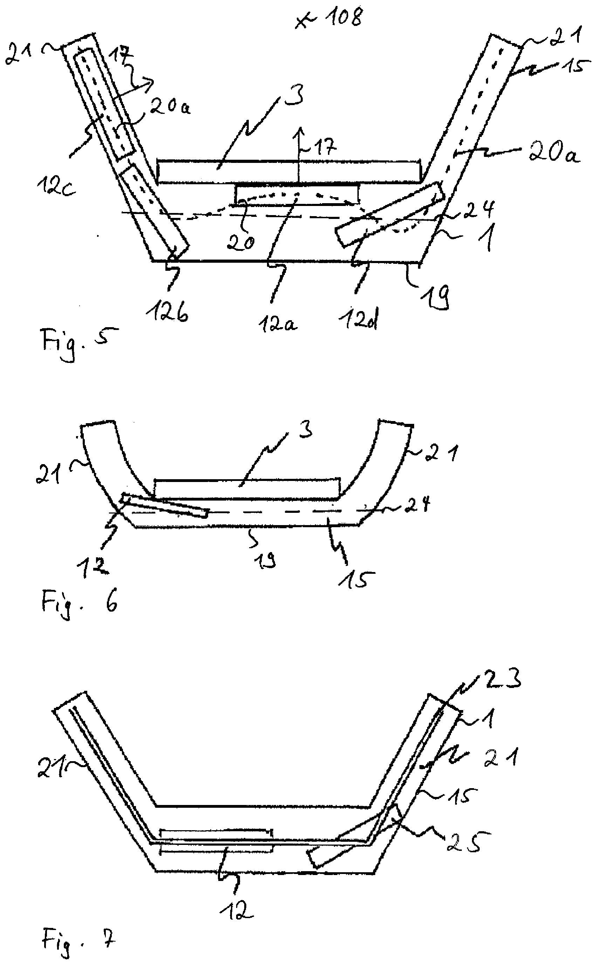

Fig. 5 zeigt in einer Fig. 4 entsprechenden Seitenansicht eine weitere Ausführungsform der Detektoranordnung 1. Die Detektoranordnung 1 ist wiederum unterhalb der Auflage 3 angeordnet, allerdings sind die Seitenelemente 21 zwar gegenüber dem mittleren Teil 19 abgewinkelt, aber fest mit dem mittleren Teil 19 verbunden, also nicht abklappbar. Der Detektor 12 ist auf einer Abbildungsfläche 20, die sich entlang der Detektoranordnung 1 erstreckt, parallel zu dem Schienensystem 23 geführt. In den Seitenelementen 21 ist die Abbildungsfläche 20 als Seitenabbildungsfläche 20a, die gegenüber der Abbildungsfläche 20 abgewinkelt ist, weitergeführt. Die Abbildungsfläche 20 sowie die Seitenabbildungsfläche 20a können beliebige Formen aufweisen, beispielsweise Ebenen oder Sattelflächen sein. Gegenüber der Abbildungsfläche 20 in dem mittleren Teil 19 der Detektoranordnung 1 sind die Seitenelemente 21 um 75° abgewinkelt.

Die Abbildungsfläche 20, die sich durch den mittleren Teil 19 und die beiden Seitenelemente 21 erstreckt, ist gekrümmt und der Detektor 12 kann auf der Abbildungsfläche 20 bzw. der Seitenabbildungsfläche 20a in mehrere zu der Auflage 3 und dem abzubildenden Objekt raumfeste Aufnahmepositionen 12a, 12b, 12c und 12d bewegt werden. Der Detektor 12 kann hierzu in dem mittleren Teil 19 auf der Abbildungsfläche 20 in mehrere Aufnahmepositionen wie beispielsweise die Aufnahmeposition 12a bewegt werden. Alle der Aufnahmepositionen auf dem mittleren Teil 19 können in weiteren Ausführungsformen parallel zu der Auflage 3 liegen, d. h. der Detektor 12 ist in diesen Fällen ebenfalls parallel zu der Auflage 3 ausgerichtet. Durch eine Drehung des Detektors 12 ist dieser in die Seitenelemente 21 verfahrbar, wie in den Aufnahmepositionen 12d und 12b dargestellt. Die Aufnahmeposition 12c kennzeichnet einen Anschlagspunkt in dem Seitenelement 21, d. h. der Detektor 12 kann nicht mehr weiter bewegt werden. In dieser Aufnahmeposition 12c befindet sich der Detektor 12 komplett oberhalb der Auflage 3 und oberhalb einer Mittelebene 24, die mittig zwischen zwei Oberflächen des Gehäuses 15 des mittleren Teils 19 angeordnet ist und in weiteren Ausführungsformen der Abbildungsfläche 20 des mittleren Teils 19 entspricht. Die Abbildungsfläche 20 wird nicht von dem Detektor 12 geschnitten, da der Detektor 12 mit sämtlichen seiner Bestandteile oberhalb der der Abbildungsfläche 20 liegt. Lediglich die gegenüber der Abbildungsfläche 20 abgewinkelte Seitenabbildungsfläche 20a wird von dem Detektor 12 in der Aufnahmeposition 12c tangiert.

In weiteren Ausführungsformen kann die Detektoranordnung 1 auch U-förmig oder halbrund sein. In der halbrunden Form weist die Oberflächennormale 17 des Detektors 12 in jeder Position auf eine zentrale Achse 108, die bevorzugt parallel zu einer Längsachse der Detektoranordnung 1 liegt. In Fig. 5 ist die zentrale Achse 108 als Schnittpunkt der Oberflächennormalen 17 in den Aufnahmepositionen 12a und 12c eingezeichnet, Die zentrale Achse 108 verläuft bevorzugt durch einen Mittelpunkt des abzubildenden Objekts, da der Abstand zu diesem Mittelpunkt in den entsprechenden Aufnahmepositionen gleich groß ist und somit eine Weiterverarbeitung der aufgenommenen Projektionsbilder erleichtert wird.

Eine weitere Ausführungsform der Detektoranordnung 1 mit gegenüber dem mittleren Teil 19 gekrümmten Seitenelementen 21 ist in Fig. 6 dargestellt. Durch die Krümmung der Seitenelemente 21 befindet sich in einem von den Seitenelementen 21 und dem mittleren Teil 19 umschlossenen Innenraum der Detektoranordnung 1 mehr Raum für das abzubildende Objekt. Die Seitenelemente 21 weisen hierbei eine Breite auf, die kleiner als eine Breite des mittleren Teils 19 ist. In weiteren Ausführungsformen können die Breiten jedoch auch gleich groß sein oder die Breite der Seitenelemente 21 sogar größer als die Breite des mittleren Teils 19 sein. Eine Breite der Auflage 3 ist identisch zu der Breite des mittleren Teils 19.

In Fig. 7 ist in einer Fig. 5 entsprechenden Ansicht ein weiteres Ausführungsbeispiel der Detektoranordnung 1 gezeigt. Innerhalb des Gehäuses 15 der Detektor 12 auf dem Schienensystem 23 als Positioniervorrichtung 13 geführt, das sich durch den mittleren Teil 19 und die Seitenelemente 21 zieht. Das Schienensystem 23 ist linienförmig und weist keine Krümmung auf, allerdings sind die in den Seitenelementen 21 befindlichen Abschnitte des Schienensystems 23 gegenüber dem in dem mittleren Teil 19 verlaufenden Abschnitt abgewinkelt. Zusätzlich zu dem Detektor 12 befindet sich nun ein weiterer Detektor 25 in der Detektoranordnung 1. Der weitere Detektor 25 kann hierbei baugleich zu dem Detektor 12 sein, aber auch andere Abmessungen aufweisen oder einen anderen Detektortyp umfassen. Der Detektor 12 und der weitere Detektor 25 sind in dem in Fig. 7 gezeigten Beispiel auf dem gleichen Schienensystem 23 geführt, allerdings können die beiden Detektoren 12, 25 auch auf verschiedenen Schienensystemen geführt sein. Außerdem wird der Detektor 12 entweder gekoppelt mit dem weiteren Detektor 25 oder frei zu diesem bewegt. Statt eines Schienensystems kann auch ein Linearantrieb zum Bewegen verwendet werden. Eine Recheneinheit kann automatisiert die Detektoren 12, 25 über Motoren bewegen und vorgeben, an welcher Position die Detektoren 12, 25 am vorteilhaftesten positioniert werden. Das Gehäuse 15 kann auch als Auflage 3 dienen, d. h. das abzubildende Objekt kann auch ohne die ansonsten benötigte Auflage 3 direkt auf das Gehäuse 15 gelegt werden und auf diesem liegend abgebildet werden.

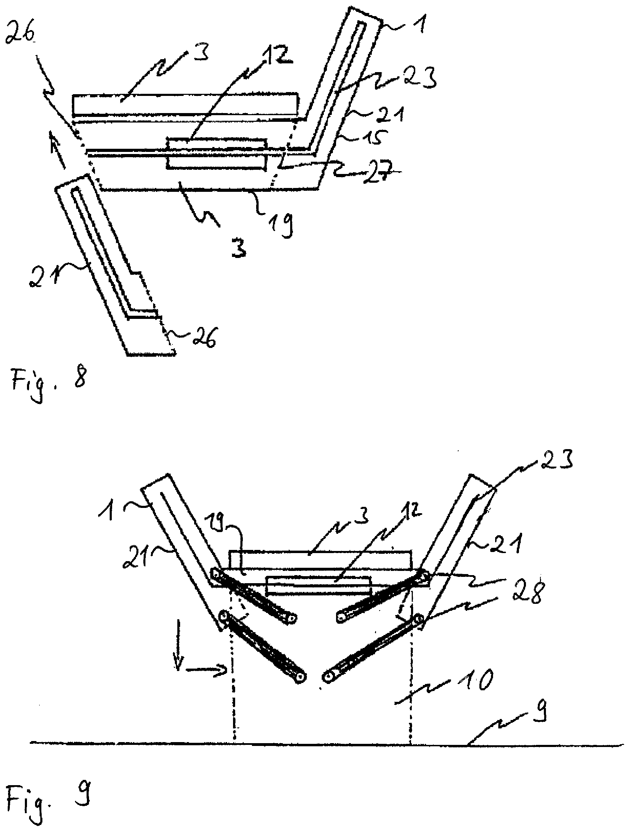

Fig. 8 zeigt in einer Fig. 5 entsprechenden seitlichen Ansicht eine Ausführungsform der Detektoranordnung 1, bei der die Seitenelemente 21 von dem mittleren Teil 19 abnehmbar sind. Hierfür sind an den Seitenelementen 21 und dem mittleren Teil 19 jeweils ein offener Verbindungsbereich 26 angeordnet, der nach dem Verbinden der Seitenelemente 21 und des mittleren Teils 19 einen von dem Gehäuse 15 umschlossenen geschlossenen Verbindungsbereich 27 bildet.

In Fig. 9 ist ein weiteres Ausführungsbeispiel der Detektoranordnung 1 in einer Fig. 3 entsprechenden Seitenansicht mit in ihrer Bewegung geführten Seitenelementen 21 gezeigt. Die Detektoranordnung 1 ist über den Standfuß 10 oder einen Gehäusecontainer mit dem Boden 9 verbunden. An dem Standfuß 10 befinden sich Führungsstäbe 28, die die Seitenelemente 21 in ihrer durch Pfeile schematisch angedeuteten Bewegung während des Anklappens oder Abklappens führen. Im angeklappten Zustand ist das Schienensystem 23 durchgängig durch den mittleren Teil 19 und die beiden Seitenelemente 21.

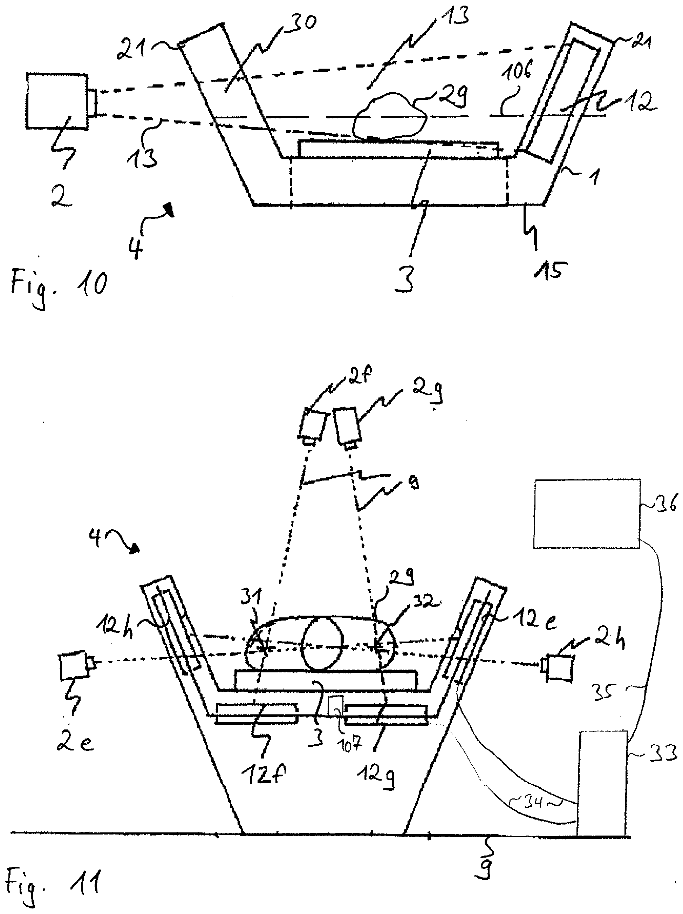

In einer Fig. 5 entsprechenden Ansicht ist eine Ausführungsform der Röntgenvorrichtung 4, bei der der Detektor 12 und die Röntgenquelle 2 in mehrere Aufnahmepositionen bewegbar sind, in Fig. 10 gezeigt. Die Röntgenquelle 2 ist in diesem Ausführungsbeispiel seitlich zu der Detektoranordnung 1 positioniert. Der Detektor 12 ist in den von der Röntgenquelle 2 weiter entfernten Seitenelement 21 bewegt, sodass der Röntgenkegeistrahl 14 zunächst das Material 30 des ersten der Seitenelemente 21, anschließend das auf der Auflage 3 befindliche abzubildende Objekt 29 durchstrahlt und schließlich auf den in dem zweiten der Seitenelemente 21 befindlichen Detektor 12 trifft. Durch die in Fig. 10 gezeigte Anordnung der Röntgenvorrichtung 4 können große Öffnungswinkel für eine Röntgenbildgebung erreicht werden. In der in Fig. 10 gezeigten Aufnahmeposition des Detektors 12 ragt dieser zumindest teilweise über eine durch einen Mittelpunkt des abzubildenden Objekts 29 verlaufende Ebene 106, die parallel zu einer Oberfläche der Auflage 3 und des mittleren Teils 19 der Detektoranordnung 1 liegt, hinaus.

In der in Fig. 11 in einer Fig. 10 entsprechende Ansicht gezeigten Ausführungsform der Röntgenvorrichtung 4 sind sowohl der Detektor 12 als auch die Röntgenquelle 2 in mehreren Positionen gezeigt. Zum Aufnehmen von Projektionsbildern des abzubildenden Objekts 29, das in dem in Fig. 11 gezeigten Beispiel ein auf der Auflage 3 liegender Patient ist, werden die Röntgenquelle 2 und der Detektor 12 vorzugsweise gleichzeitig in verschiedene Aufnahmepositionen bewegt. In einer Aufnahmeposition 2e der Röntgenquelle 2 bzw. einer korrespondierenden Aufnahmeposition des Detektors 12e befindet sich die Röntgenquelle 2 und der Detektor 12 in den in Fig. 10 bereits dargestellten Positionen. In einer Aufnahmeposition 2f der Röntgenquelle 2 bzw. einer Aufnahmeposition des Detektors 12f befindet sich die Röntgenquelle 2 oberhalb des abzubildenden Objekts 29, während der Detektor 12 unterhalb der Auflage 3 unter einer linken Hälfte des abzubildenden Objekts 29 der Röntgenquelle 2 gegenüber liegt. In einer Aufnahmeposition 2g befindet sich die Röntgenquelle 2 ebenfalls oberhalb des abzubildenden Objekts 29, während der Detektor 12 in eine unterhalb des abzubildenden Objekts 29 befindliche Aufnahmeposition 12g bewegt wurde. In einer Aufnahmeposition 2h bzw. 12h sind die Röntgenquelle 2 und der Detektor 12 spiegelbildlich zu den Aufnahmepositionen 2e und 12e angeordnet.

In den verschiedenen Aufnahmepositionen gemachte Projektionsbilder des ersten Zielgebiets 31 und des zweiten Zielgebiets 32 des abzubildenden Objekts 29 können zusammengeführt werden, um ein dreidimensionales Modell des jeweiligen Zielgebiets 31, 32 durch eine Recheneinheit 33 als Auswerteeinheit zu rekonstruieren. Hierzu ist die Recheneinheit 33 mit dem Detektor 12 über ein Kabel 34 verbunden. Alternativ können statt über das Kabel 34 die Projektionsbilder auch kabellos, beispielsweise über eine Funkverbindung, von dem Detektor 12 an die Recheneinheit 33 übermittelt werden. Die Recheneinheit 33 wertet die Projektionsbilder aus und berechnet die Rekonstruktion des untersuchten Zielgebiets. Die Rekonstruktion wird entweder durch ein weiteres Kabel 35 oder kabellos an eine Ausgabeeinheit 36, im dargestellten Ausführungsbeispiel einen Bildschirm, übermittelt und auf der Ausgabeeinheit 36 angezeigt. Die Ausgabeeinheit 36 kann sich hierfür auch direkt an der Detektoranordnung 1 befinden. Bei mehrfach durch verschiedene Projektionsaufnahmen abgebildeten Zielgebieten können einzelne Projektionsaufnahmen auch für mehrere Zielgebiete verwendet werden. In dem in Fig. 11 gezeigten Beispiel wird das Zielgebiet 32 in den Aufnahmepositionen 2e, 2g und 2h von von der Röntgenquelle 2 ausgesandter Röntgenstrahlung durchstrahlt. Bei einem Zielgebiet, das größer als der verwendete Detektor 12 ist, können auch mehrere Projektionsaufnahmen zu einer zusammenhängenden Projektionsaufnahme zusammengesetzt werden. Zum Bewegen des Detektors 12 ist in der Positioniervorrichtung 13 unterhalb der Auflage 3 in dem mittleren Teil 19 der Detektoranordnung 1 ein Motor 107 vorgesehen, der über ein Kabel mit der Recheneinheit 33 verbunden ist und von dieser angesteuert die Bewegung des Detektors 12 veranlasst.

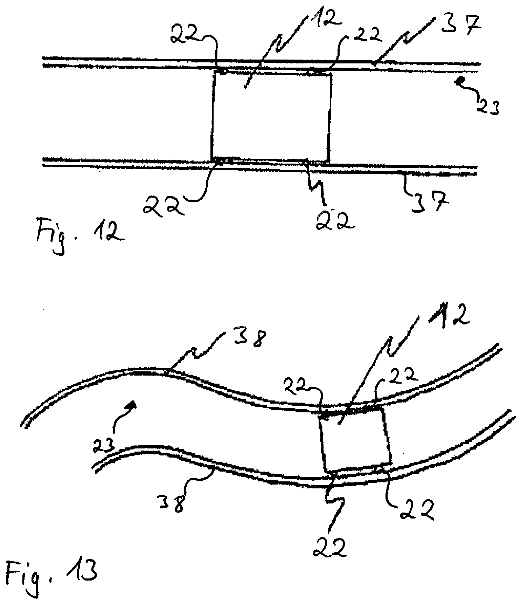

Fig. 12 zeigt eine Draufsicht auf ein geradliniges Schienensystem 23 der Detektoranordnung 1 wie es den zuvor erläuterten Ausführungsbeispielen zum Einsatz kommen kann. Der Detektor 12 ist über das beidseitig an dem Detektor 12 angebrachte Bewegungslager 22, das im dargestellten Ausführungsbeispiel ein Schienenlager ist, auf dem Schienensystem 23 gelagert. Je eine gerade Schiene 37 des Schienensystems 23 verläuft oberhalb und unterhalb des Detektors 12. Je zwei der Bewegungslager 22 sind auf einer der beiden geraden Schienen 37 geführt. Die Bewegungslager 22 wie auch die geraden Schienen 37 sind aus Plastik, können in weiteren Ausführungsbeispielen aber auch verschiedenste Materialien wie Metall oder kohlestofffaserverstärkte Kunststoffe wie Karbon umfassen.

Eine Fig. 12 entsprechende Anordnung von Schienensystem 23 und Detektor 12 ist in Fig. 13 ebenfalls in Draufsicht gezeigt. Im Gegensatz zu dem in Fig. 12 gezeigten Ausführungsbeispiel ist der Detektor 12 über die Bewegungslager 22 nun jedoch nicht auf geraden Schienen 37 sondern auf gebogenen Schienen 38 geführt, sodass das Schienensystem 23 einen gekrümmten Verlauf aufweist.

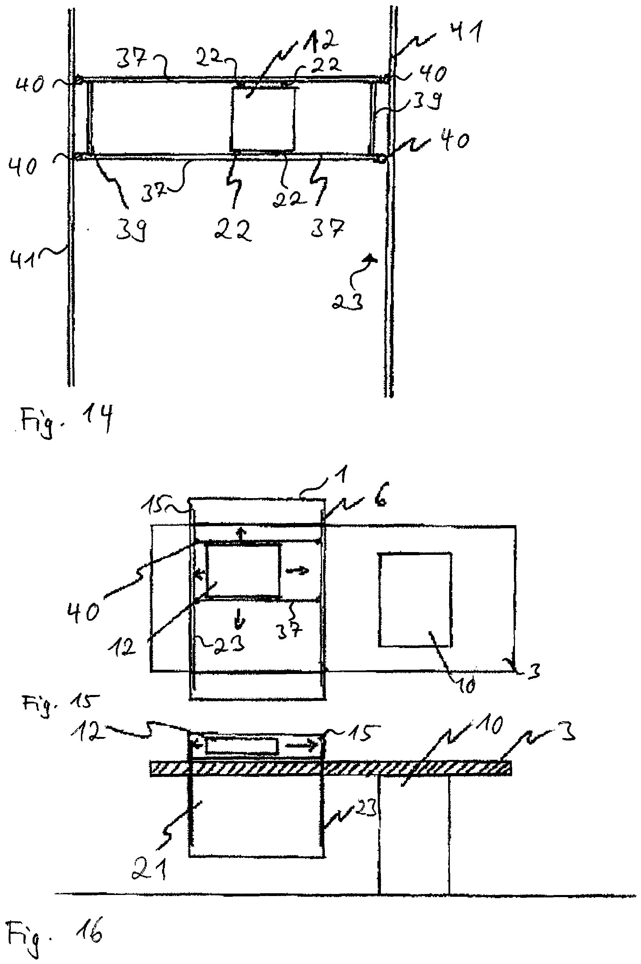

Das Schienensystem 23 kann auch, wie in Fig. 14 in einer Fig. 12 entsprechenden Draufsicht gezeigt, kaskadiert aufgebaut sein. Ein Mittelteil des kaskadierten Schienensystems 23 entspricht in seinem Aufbau dem in Fig. 12 dargestellten System. Diese Anordnung wird durch zwei Streben39 gestützt und über jeweils zwei an beiden Enden in Nachbarschaft zu einer der Streben 39 angeordneten weiteren Schienenlager 40 auf weiteren geraden Schienen 41 geführt. Statt gerader Schienen 37, 41 können natürlich auch gebogene oder freigeformte Schienen verwendet werden. Je zwei der weiteren Schienenlager 40 sind hierbei auf einer der weiteren geraden Schienen 41 verfahrbar. Durch eine Überlagerung mehrerer Schienensysteme kann der Detektor 12 nahezu beliebig in verschiedene Raumrichtungen verfahren werden. Raumachsen, um die eine Bewegung mittels des dargestellten kaskadierten Schienensystems 23 erfolgt, liegen hierbei orthogonal zueinander in einer Ebene.

Fig. 15 zeigt in Draufsicht eine Ausführungsform der Detektoranordnung 1, bei der der Detektor 12 oberhalb der Auflage 3 verfahrbar ist. Die Auflage 3 ist über den Standfuß 10 oder einen Tischfuß mit dem Boden 9 verbunden. Auf der Auflage 3 ist die Detektoranordnung 1 angebracht. Innerhalb des Gehäuses 15 der Detektoranordnung 1 ist der Detektor 12 auf dem in Fig. 14 dargestellten Schienensystem 23 geführt und kann in die durch Pfeile angedeutete Richtungen bewegt werden. In alternativen Ausführungsformen kann die gezeigte Detektoranordnung 1 auch unterhalb der Auflage 3 angebracht sein oder einen Teil der Auflage 3 bilden. Die Detektoranordnung 1 weist abklappbare Seitenelemente 21 auf, die aus einer unterhalb der Auflage 3 befindlichen Position aufgestellt werden können und nach Aufnahmen wieder abgesenkt werden. Das Anklappen und Abklappen der Seitenelemente 21 kann in dieser, wie auch in den bereits vorgestellten Ausführungsformen entweder manuell oder automatisiert erfolgen, wobei das automatisierte Verfahren durch die Recheneinheit 33 als Steuereinheit gesteuert werden kann.

In Fig. 16 ist das in Fig. 15 gezeigte Ausführungsbeispiel in einer seitlichen Ansicht dargestellt. Der Detektor 12 ist oberhalb der Auflage 3 angeordnet, kann allerdings auch in die seitlich der Auflage 3 abgeklappten Seitenelemente 21 bewegt werden. Hierzu befindet sich in den Seitenelementen 21 auch ein Teil der Schienensystems 23.

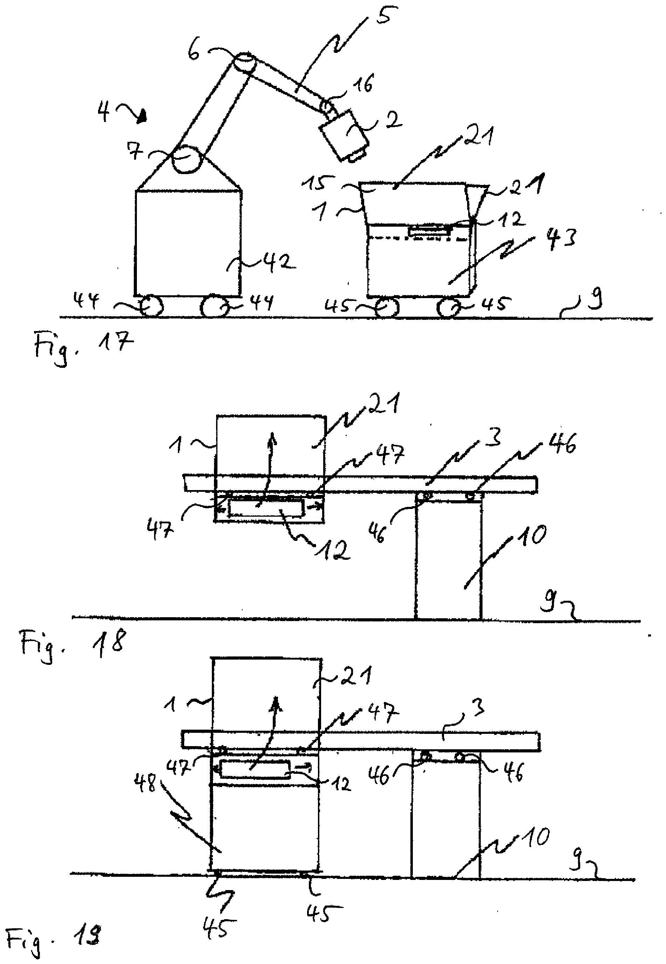

Fig. 17 zeigt in einer Seitenansicht eine Ausführungsform der Röntgenvorrichtung 4, bei der sowohl die Detektoranordnung 1 als auch die Röntgenquelle 2 mobil angeordnet sind. Die Röntgenquelle 2 ist an dem Roboterarm 5 anbracht, der über das erste Gelenk 6, das zweite Gelenk 7 und das dritte Gelenk 16 beweglich ist. Der Roboterarm 5 ist auf einem Wagen 42 befestigt, der über Räder 44 oder auf einem auf dem Boden 9 angeordneten Schienensystem beweglich ist. Die Detektoranordnung 1 hat aufgestellte Seitenelemente 21 und ist auf einem Detektorgehäusecontainer 43 montiert, der ebenfalls auf Rädern 45 oder einem auf dem Boden 9 angeordneten Schienensystem verfahrbar ist. Das Gehäuse 15 der Detektoranordnung 1 dient zugleich auch als die Auflage 3, auf der das abzubildende Objekt 29 aufgelegt wird. Eine räumliche Zuordnung und bzw. oder Lokalisierung der Röntgenquelle 2 und des Detektors 12 zueinander kann durch Marker erfolgen, die an einem der beiden Bauelemente angebracht sind, wobei das jeweils andere Bauelement, als dasjenige ohne angebrachte Marker, eine Auswerteeinheit zum Auswerten der räumlichen Position umfasst.

In Fig. 18 ist in einer seitlichen Ansicht eine weitere Ausführungsform der an der Auflage 3 befestigten Detektoranordnung 1 gezeigt, bei der der Detektor 12 unterhalb der Auflage 3 verfahrbar ist. Die Auflage 3 ist über zwei zwischen der Auflage 3 und dem Standfuß 10 angeordnete Bewegungslager 46 über den Standfuß 10 bewegbar. Die Detektoranordnung 1 mit angeklappten Seitenelementen 21 ist über zwei Bewegungslager 47 verschiebbar an der Auflage 3 befestigt. Der Detektor 12 kann somit eine Bewegung innerhalb der Positioniervorrichtung 13 der Detektoranordnung 1 vollziehen und eine zweite Bewegung mit der Detektoranordnung 1 an der Auflage 3. Hierbei kann sich der Detektor 12 nicht nur translatorisch bewegen, sondern auch gedreht werden. In weiteren Ausführungsbeispielen kann auch der Standfuß 10 beweglich auf dem Boden 9 angeordnet sein. Dies kann insbesondere dann erfolgen, wenn die Detektoranordnung 1 mobil oder stationär mit dem Boden 9, einer Wand oder der Decke 8 verbunden ist.

Fig. 19 zeigt eine Fig. 18 entsprechende Darstellung einer weiteren Ausführungsform der an der Auflage 3 verschiebbar befestigten Detektoranordnung 1. Im Gegensatz zu Fig. 19 ist die Detektoranordnung 1 nun nicht nur an der Auflage 3 verschiebbar befestigt, sondern über den Detektorcontainer 48 und die Räder 45 auch auf dem Boden 9 verschiebbar. Ansonsten entspricht das in Fig. 19 gezeigte Ausführungsbeispiel dem bereits in Fig. 18 beschriebenen Beispiel. Die Seitenelemente 21 sind wiederum angeklappt und befinden sich oberhalb der Auflage 3.

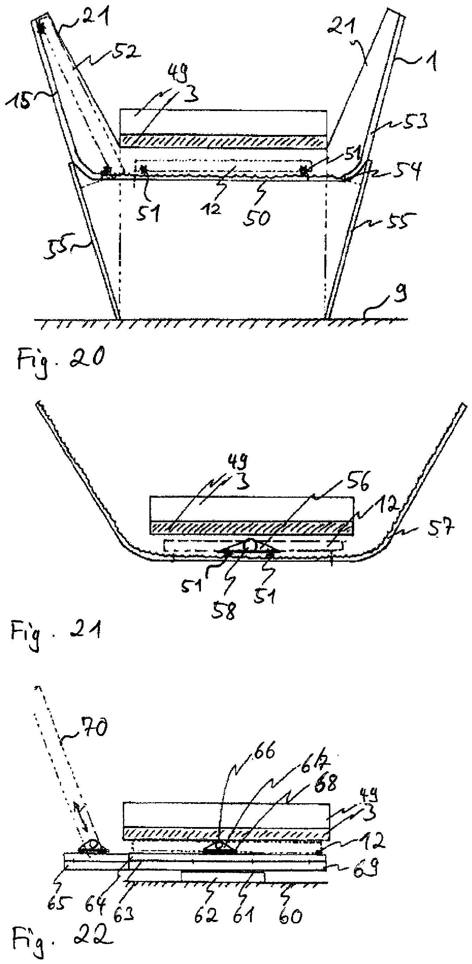

Eine Fig. 3 entsprechende Darstellung der Detektoranordnung 1 mit Führung durch einen Zahntrieb 51 ist in Fig. 20 dargestellt. Die Detektoranordnung 1 ist wiederum unterhalb der Auflage 3 angeordnet. Die Auflage 3 ist nun mit einer Schutzlage 49 wie einem Polster versehen, die, beispielsweise bei Verschmutzung, einfach abgezogen und ausgewechselt werden kann. Die Schutzlage 49 ist aus einem für Röntgenstrahlung durchlässigen Material. Der Detektor 12 ist über zwei gleichlaufende Zahntriebe 51 auf einer Zahnstange 50 als Positioniervorrichtung 13 geführt. Die zwei Zahntriebe 51 sind jeweils an einem Ende des Detektors 12 angebracht. Über eine Fügestelle 54 zwischen der Zahnstange 50 und einer Zahnradgleitführung 53 in den Seitenelementen 21 ist der Detektor 12 auf der Zahnstange 50 in die Seitenelemente 21 verschiebbar. Der Detektor 12 ist in einer Aufnahmeposition 52 in einem Seitenelement 21 dargestellt, wobei einer der beiden Zahntriebe 51, der von dem jeweiligen Seitenelement 21 abgewandt ist, noch auf der Zahnstange 50 ruht und zum Bewegen in diese greift, während der andere der beiden Zahntriebe 51 auf der Zahnradgleitführung 53 auf einem unteren Teil des Gehäuses 15 aufliegt. Unterhalb jedes der Seitenelemente 21 ist jeweils eine Seitenwand 55 angeordnet, die bündig mit dem Seitenelement abschließt und einen unterhalb der Detektoranordnung 1 und oberhalb des Bodens 9 liegenden Raum begrenzt.

Fig. 21 zeigt in einer Figur 20 entsprechenden Ansicht eine Ausführungsform der Detektoranordnung 1, die der in Fig. 20 gezeigten entspricht, bei der jedoch eine durchgehende gebogene Zahnstange 57 verwendet wird, auf der der Detektor 12 durch einen Schlitten 56 geführt wird. Die gebogene Zahnstange 57 ist in einem parallel zu der Auflage 3 angeordneten mittleren Teil 19 sowie in den gegenüber diesem mittleren Teil 19 abgewinkelten seitlichen Teilen gerade, jedoch im Übergangsbereich zwischen den Teilen gebogen. Der Schlitten 56 weist an seiner Unterseite zwei gleichlaufende Zahntriebe 51 auf, die als Zahnräder ausgeführt sind. Der Schlitten 56 ist über eine angetriebene Rotationsachse 58 mit dem Detektor 12 verbunden und verfährt den Detektor 12 durch Zahneingriffe der Zahntriebe 51 in die gebogene Zahnstange 57. Diese lineare Bewegung kann mit einer Drehung bzw. Anstellung des Detektors 12 um die Rotationsachse 58 kombiniert werden.

Die in Fig. 21 dargestellte Detektoranordnung 1 ist in Fig. 22 ebenfalls in einer Seitenansicht widergegeben, unterscheidet sich nun jedoch durch eine Linearführung 68, die das Schienensystem 23 oder die Zahnstange 50 überflüssig macht.

Fig. 22 zeigt eine Fig. 21 entsprechende Darstellung der Detektoranordnung 1 aus Fig. 21 mit einem Linearantrieb 64. Eine Kombination aus Linearachsen und Anstellachsen bewegt den Detektor 12 unterhalb und seitlich der Auflage 3 an die gewünschte Position, sodass ein Schienensystem 23 entfallen kann. Hierzu ist unterhalb der Auflage 3 eine bewegliche Montagefläche 60 parallel zu der Auflage 3 angeordnet. Auf der beweglichen Montagefläche 60 befindet sich ein an die bewegliche Montagefläche 60 montierter Auslenker-Schlitten 62, der über eine Führung 61, die in dem in Fig. 22 dargestellten Ausführungsbeispiel eine Kugellagerführung umfasst, mit einer Auslenkerbasis 69 verbunden. Die Auslenkerbasis 69 ist zwischen der Auflage 3 und dem Auslenker-Schlitten 62 angebracht und beweglich. Über eine Verbindung 63 sind der Auslenker und der Linearantrieb 64 miteinander in Kontakt. Zwischen der Auslenkerbasis 69 und der Auflage 3 ist ein Linearantrieb-Schlitten 67 angeordnet, der über eine Linearführung 68, die mit der Auslenkerbasis 69 und dem Detektor 12 verbunden ist. Der Linearantrieb-Schlitten 67 ist über eine Drehachse 66 beweglich. Die Auslenkbasis 69 kann durch ein Verschieben parallel zu der Auflage 3 in eine seitliche Position 65 verfahren werden, in der die Auslenkbasis 69 zumindest teilweise nicht mehr von der Auflage 3 überdeckt wird. Durch den Linearantrieb-Schlitten 67 kann in dieser Position der Detektor 12 um die Drehachse 66 des Linearantrieb-Schlittens 67 gedreht werden und somit in eine Position 70 verfahren werden, in der der Detektor 12 seitlich neben der Auflage 3 abgewinkelt zu der Auflage 3 angeordnet ist.

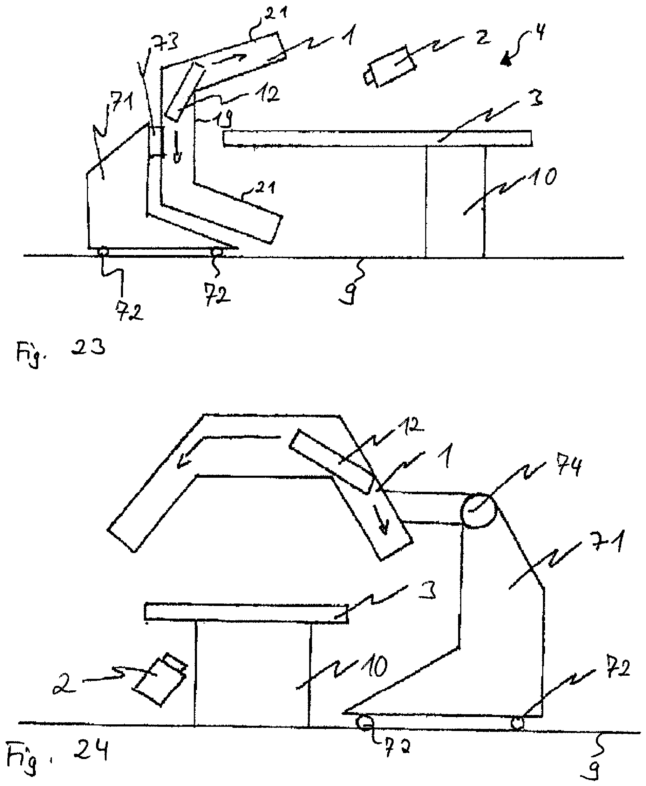

In Fig. 23 ist in einer seitlichen Ansicht eine Ausführungsform der Röntgenvorrichtung 4 gezeigt, bei der die Detektoranordnung 1 an einem bewegbaren Wagen 71 rotierbar angebracht ist. Die Auflage 3 ist über den Standfuß 10 mit dem Boden 9 verbunden, allerdings ist die Detektoranordnung 1 nicht mehr an der Auflage 3 befestigt. Die Detektoranordnung 1 entspricht in ihren Merkmalen in dem in Fig. 23 dargestellten Ausführungsbeispiel der in Fig. 5 beschriebenen Detektoranordnung 1 und ist über eine Drehachse 73 an dem Wagen 71 befestigt. Der Wagen 71 ist über Wagenräder 72 oder alternativ über ein Schienensystem auf dem Boden 9 geführt, sodass durch Bewegen des Wagens 71 die Detektoranordnung 1 frei, jedoch raumfest bezüglich der Auflage 3 positioniert werden kann. Über die Drehachse 73 kann die Detektoranordnung 1 gedreht werden, sodass beispielsweise die Seitenelemente 21 mit dem mittleren Teil 19 in einer Ebene mit der Auflage 3 liegen. Durch die ebenfalls frei bezüglich der Auflage 3 bewegliche Röntgenquelle 2, die in dem in Fig. 23 gezeigten Beispiel exemplarisch ober halb der Auflage 3 angeordnet ist, können somit Projektionsaufnahmen aus mehreren Winkeln gemacht und durch die in Fig. 23 nicht dargestellte Auswerteeinheit ausgewertet und die Ausgabeeinheit dargestellt werden.

In Fig. 24 ist eine weitere Ausführungsform der Röntgenvorrichtung 4 aus Fig. 23 in einer Fig. 23 entsprechenden seitlichen Ansicht dargestellt. Die Röntgenquelle 2 ist nun unterhalb der Auflage 3 angeordnet, während die Detektoranordnung 1 und somit der Detektor 12 oberhalb der Auflage 3 liegen. Der Wagen 71 verfügt statt der Drehachse 73 über eine Klapp-/Drehachse 74, die den Wagen 71 mit der Detektoranordnung 1 verbindet. Durch die Klapp-/Drehachse 74 kann die Detektoranordnung 1 durch Klappen oberhalb der Auflage 3 positioniert werden. Die Detektoranordnung 1 und somit die Positioniervorrichtung 13 sowie der Detektor 12 sind oberhalb, unterhalb und seitlich zu der Auflage 3 verfahrbar.

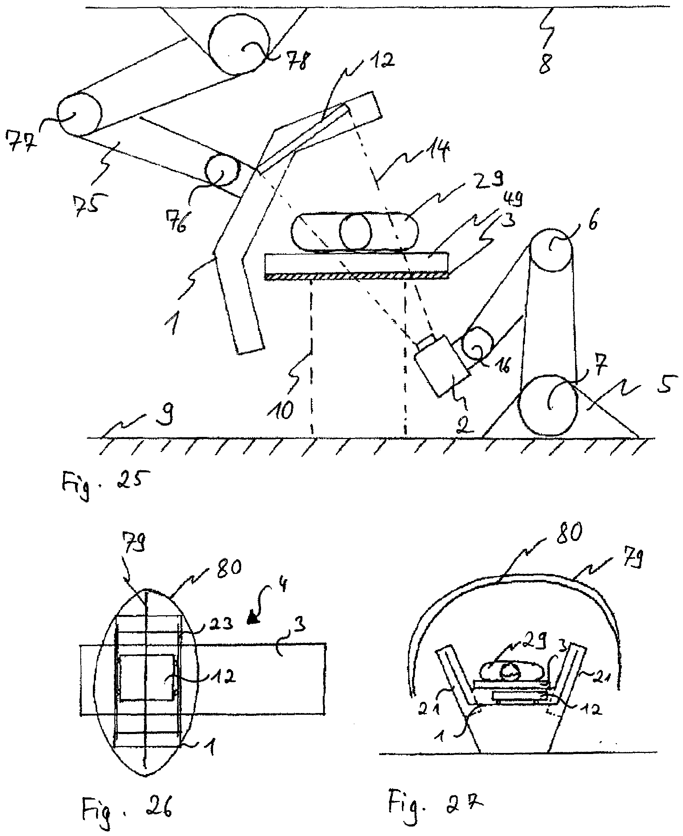

Fig. 25 stellt in einer seitlichen Ansicht eine weitere Ausführungsform der Röntgenvorrichtung 4 dar, bei der die Röntgenquelle 2 und die Detektoranordnung 1 jeweils an Roboterarmen 5, 75 befestigt sind und durch die Roboterarme 5, 75 frei durch die Recheneinheit 33 positionierbar sind. Die Auflage 3 ist wiederum über den Standfuß 10 mit dem Boden 9 verbunden, an dem auch der die Röntgenquelle 2 tragende Roboterarm 5 befestigt ist. Dieser Roboterarm 5 kann die Röntgenquelle 2 durch das erste Gelenk 6, das zweite Gelenk 7 und das dritte Gelenk 16 frei positionieren. Die Detektoranordnung 1 und somit auch der Detektor 12 sind über den Roboterarm 75 mit der Decke 8 verbunden. Der Roboterarm 75 kann alternativ aber natürlich auch an dem Boden 9 befestigt sein. Der Roboterarm 75 bewegt die Detektoranordnung 1 über ein erstes Gelenk 76, ein zweites Gelenk 77 und ein drittes Gelenk 78, sodass die Detektoranordnung 1 in sechs Freiheitsgraden, drei rotatorischen und drei translatorischen, einstellbar ist. Zusätzlich kann der Detektor 12 innerhalb der Detektoranordnung 1 bewegt werden, sodass der Röntgenkegelstrahl 14 der Röntgenquelle 2 auf das abzubildende Objekt 29 durchdringt und auf den Detektor 12 auftrifft.

Fig. 26 zeigt eine Draufsicht auf die in Fig. 1 dargestellte Röntgenvorrichtung 4 mit Bewegungsbahnen der Röntgenquelle 2. Die Detektoranordnung 1 ist auf der Auflage 3 befestigt, wobei der Detektor 12 auf dem Schienensystem 23 wie bereits beschrieben verfahrbar ist. Zum Aufnehmen der Projektionsbilder wird eine kreisförmige Aufnahmebahn 79 aus vordefinierten Positionen von der Röntgenquelle 2 durchfahren. In dem in Fig. 26 gezeigten Beispiel ist die kreisförmige Aufnahmebahn 79 halbkreisförmig oberhalb der Auflage 3, umfasst jedoch einen Winkelbereich von mindestens 90° oberhalb der Auflage 3. Alternativ kann die Röntgenquelle 2 eine elliptische Aufnahmebahn 80 durchfahren, bei der zusätzlich zu der kreisförmigen Aufnahmebahn 79 eine laterale Auslenkung der Röntgenquelle 2 in Richtung einer Längsachse der Auflage 3 vorgenommen wird. Während die Röntgenquelle 2 eine der Aufnahmebahnen durchfährt, wird die Detektoranordnung 1 nicht bewegt, sondern verbleibt raumfest in der eingestellten Position. Stattdessen kann innerhalb der Detektoranordnung 1 der Detektor 12 auf der Positioniervorrichtung 13 bewegt werden. Die Recheneinheit 33 kann hierfür die Bewegung der Röntgenquelle 2 und des Detektors 12 steuern und kontrollieren.

Fig. 27 gibt die in Fig. 26 gezeigten Aufnahmebahnen 79, 80 in einer seitlichen Ansicht wieder. In jeder der Aufnahmepositionen wird bevorzugt eine Projektionsaufnahme gemacht, wobei zur Veranschaulichung größerer Bereiche, die auch bei vergrößertem Abstand zwischen der Röntgenquelle 2 und dem abzubildenden Objekt 29 nicht durch ein einzelnes Bild wiedergegeben werden können, eine Superposition mehrerer Aufnahmen erfolgen kann.

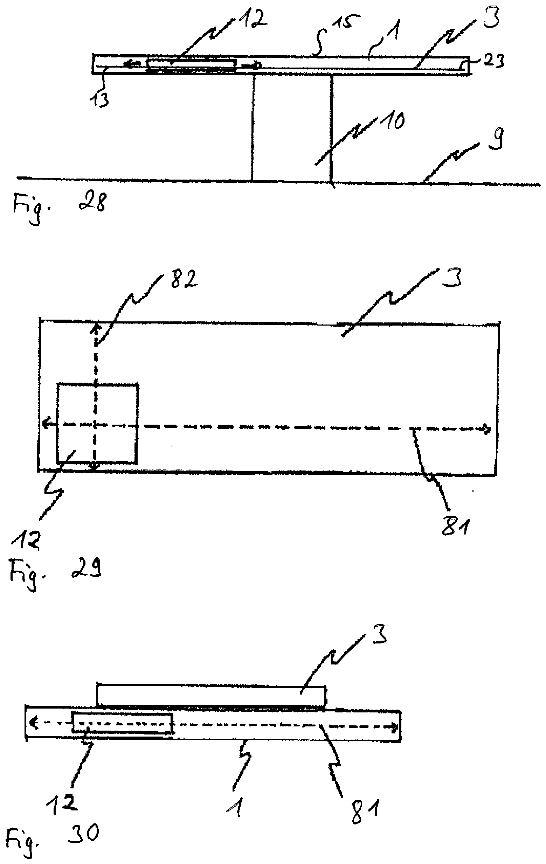

Fig. 28 zeigt eine seitliche Ansicht der Auflage 3, in die die Detektoranordnung 1 und somit die Positioniervorrichtung 13 integriert ist. Der Detektor 12 ist daher innerhalb der Auflage 3, die auch als das Gehäuse 15 dient auf dem Schienensystem 23 verfahrbar. Die Auflage 3 ist über den Standfuß 10 mit dem Boden 9 verbunden. Die in Fig. 28 wiedergegebene Anordnung ist in Fig. 29 in Draufsicht dargestellt. Der Detektor 12 ist innerhalb der Auflage 3 entlang einer Längsachse 81 der Auflage 3 über die Länge der Auflage 3 und entlang einer Querachse 82 des Auflage 3 über die Breite der Auflage 3 bewegbar. In weiteren Ausführungsformen kann der Detektor 12 auch innerhalb der durch die Längsachse 81 und die Querachse 82 definierten Ebene drehbar sein. Die Bewegung des Detektors 12 kann hierbei manuell oder durch einen Motor erfolgen.

In dem in Fig. 30 in Seitenansicht gezeigten Ausführungsbeispiel ist die Detektoranordnung 1 unterhalb, jedoch parallel zu der Auflage 3 angeordnet. Der Detektor 12 ist innerhalb der Detektoranordnung 1 wie in den Fign. 28 und 29 bereits gezeigt in zwei Raumrichtungen verschiebbar.

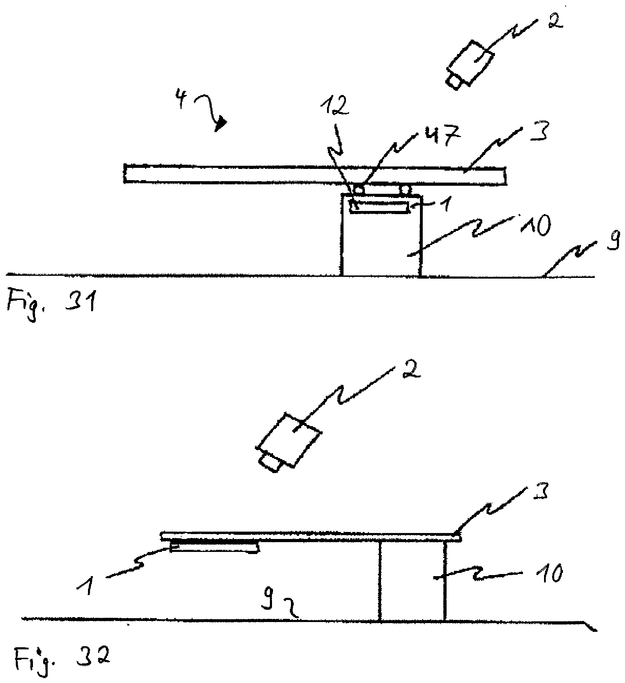

Wie in Fig. 31 in Seitenansicht dargestellt, kann in einem weiteren Ausführungsbeispiel der Röntgenvorrichtung 4 die Detektoranordnung 1 und somit der Detektor 12 in dem Standfuß 10 bzw. Tischfuß angeordnet sein. Auf dem Standfuß 10 ist die Auflage 3 über Bewegungslager 47 beweglich, sodass die Auflage 3 mit dem darauf befindlichen abzubildenden Objekt 29 relativ zu der Detektoranordnung 1 bewegt werden kann. Die Detektoranordnung 1 kann hierzu natürlich auch die bereits vorstehend beschriebenen Seitenelemente 21 aufweisen.

Ebenfalls in Seitenansicht ist in Fig. 32 ein weiteres Ausführungsbeispiel gezeigt, bei dem die Detektoranordnung 1 unmittelbar unterhalb der Auflage 3 angeordnet ist und relativ zu der Auflage 3 verfahrbar ist. Die Auflage 3 ist fest über den Standfuß 10 mit dem Boden 9 verbunden und die Röntgenquelle 2 frei positionierbar sowie von dem Boden 9 gelöst.

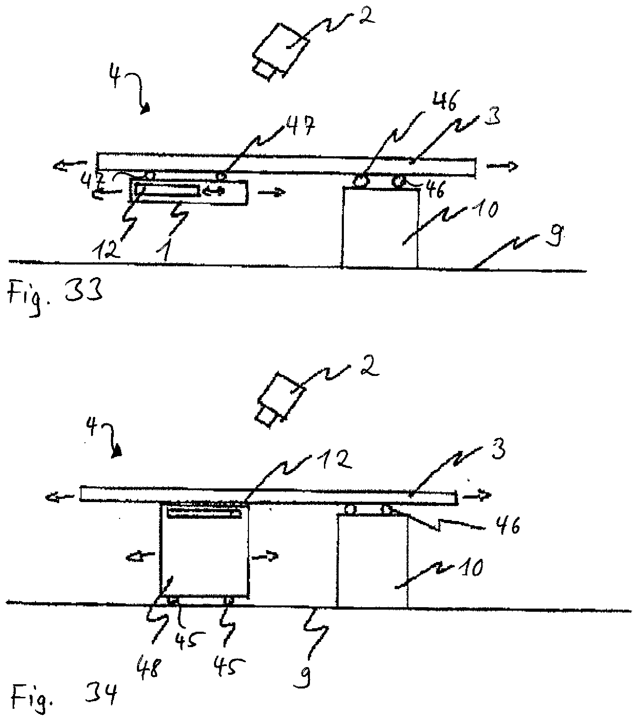

In Fig. 33 ist in einer Fig. 32 entsprechenden Ansicht ein Ausführungsbeispiel gezeigt, bei dem der Standfuß 10 fest mit dem Boden 9 verbunden ist, die Auflage 3 jedoch über die Bewegungslager 46 die Auflage 3 parallel zu dem Standfuß 10 verfahrbar ist. Außerdem ist die unterhalb der Auflage 3 angeordnete Detektoranordnung 1 über die Bewegungslager 47 mit der Auflage 3 verbunden und parallel zu dieser verfahrbar. Der Detektor 12 ist innerhalb der Detektoranordnung 1 angeordnet und in zwei Raumrichtungen parallel zu der Auflage 3 verfahrbar.

Fig. 34 zeigt in einer Fig. 33 entsprechenden Ansicht eine weitere Ausführungsform der Röntgenvorrichtung 4, bei der die Detektoranordnung 1 in einem beweglichen Gehäuse unterhalb der Auflage 3 angeordnet ist. Die Auflage 3 ist über die Bewegungslager 46 wie in Fig. 33 über den Standfuß 10 mit dem Boden 9 verbunden. Die Detektoranordnung 1 ist in einem Detektorcontainer 48 angebracht, der über Räder 45 oder ein Schienensystem auf dem Boden 9 verfahrbar ist. Der Detektorcontainer 48 kann hierzu mit der Auflage 3 verbunden sein oder unabhängig von dieser bewegt werden. Außerdem kann der Detektorcontainer 48 abklappbare bzw. abnehmbare Seitenelemente 21 aufweisen.

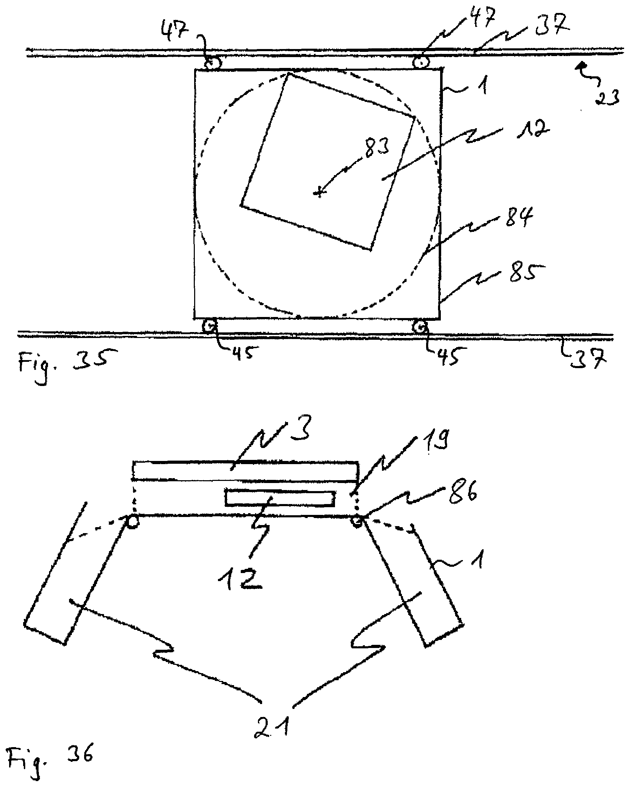

In Fig. 35 ist in Draufsicht der drehbar auf dem Schienensystem 23 gelagerte Detektor 12 gezeigt. Wie in Fig. 12 bereits dargestellte, ist der Detektor 12 ein Flachdetektor. Der Detektor 12 ist auf einer ebenen Halterung 85 geführt und diese Halterung 85 über jeweils zwei Bewegungslager 22 mit einer der geradlinigen Schienen 37 des Schienensystems 23 verbunden. Der Detektor 12 ist auf der Halterung 85 um eine mittig in der Halterung 85 befindliche Drehachse 83 drehbar. Während der Drehung durchfährt der Detektor 12 somit die kreisförmige Fläche 84, die ein Innenkreis der quadratischen Halterung 85 ist. Die Drehachse 83 kann auch an anderen Positionen der Halterung 85 angeordnet sein sowie fest oder variabel angeordnet sein. Das Schienensystem 23 kann in der Detektoranordnung 1 enthalten sein, die wie in Fig. 28 dargestellt auch direkt in die Auflage 3 eingebracht sein kann.

Fig. 36 zeigt eine Seitenansicht einer Ausführungsform der Detektoranordnung 1 mit an den mittleren Teil 19 anklappbaren Seitenelementen 21. Die Seitenelemente 21 sind beidseitig an dem mittleren Teil 19 angeordnet und über jeweils ein Scharnier 86 an diesen anklappbar oder von dem mittleren Teil 19 abklappbar, sodass die Seitenelemente 21 bündig an dem mittleren Teil 19 anliegen. Alternativ können die Seitenelemente 21 auch aus der Positioniervorrichtung 13 ausziehbar sein, indem die Seitenelemente 12 unterhalb des mittleren Teils 19 liegen und bei Bedarf herausgezogen werden. Die Seitenelemente 21 liegen in diesem Fall parallel zu der Auflage 3, unterhalb derer die Detektoranordnung 1 angeordnet ist. Im angeklappten Zustand kann der Detektor 12 von dem mittleren Teil 19 in eines der beiden Seitenelemente 21 verfahren werden. In einer weiteren Ausführungsform sind die Seitenelemente 21 auch derart an das mittlere Teil 19 anklappbar, dass die Seitenelemente 21 abgewinkelt gegenüber dem mittleren Teil 19 sind.

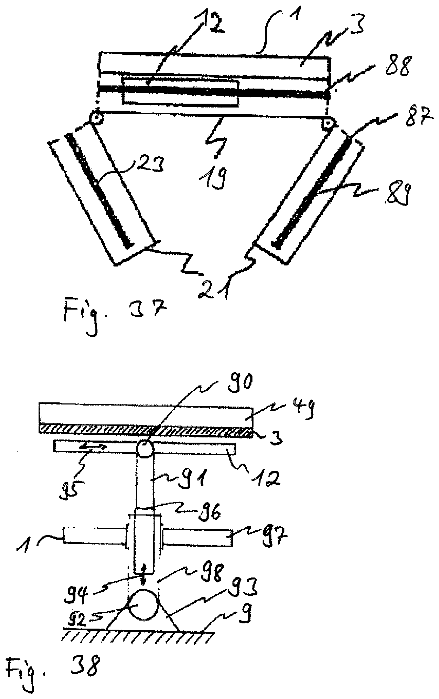

Fig. 37 zeigt die in Fig. 36 bereits dargestellte Detektoranordnung 1 mit dem Schienensystem 23 der Positioniervorrichtung. Das Schienensystem 23 umfasst eine Schiene 88 im mittleren Teil 19, ein Verbindungsstück 87 zum Schaffen einer durchgängigen Schiene beim Hochklappen der Seitenelemente 21 und eine Schiene 89, die jeweils in einem der Seitenelemente 21 liegt. Durch das Anklappen der Seitenelemente 21, die abgeklappt unterhalb des mittleren Teils 19 liegen, wird somit eine durchgehende Schiene geschaffen, auf der der Detektor 12 in die Seitenelemente 21 und der mittlere Teil 19 verschiebbar ist.

Fig. 38 zeigt eine seitliche Ansicht einer Ausführungsform der Detektoranordnung 1, bei der der Detektor 12 durch einen Hubantrieb 98 und einen Rotationsantrieb positionierbar ist. Der Detektor 12 ist um eine Rotationsachse 90 drehbar an einer U-förmigen Halterung 91 des Hubantriebs 98 gelagert. Außerdem kann der Detektor 12 entlang der Rotationsachse 90 eine zusätzliche Linearbewegung 95 parallel zu der Auflage 3 durchführen. Die U-Halterung 91 des Hubantriebs 98 ist über einen Flansch 96 mit dem eigentlichen HubAntrieb 98 verbunden und in einer vertikalen Hubbewegung 94 beweglich. Zum Anstellen des Hubantriebs 98 kann dieser an einer Linearachse 97 gelagert sein, die parallel zu dem Boden 9 verläuft. Der Hubantrieb 98 ist über eine Bodenbefestigung 93 mit dem Boden 9 verbunden. An der Bodenbefestigung 93 kann auch eine weitere Rotationsachse 92 vorgesehen sein, die ebenfalls dem Anstellen des Hub-Antriebs 98 dient.

Bei der in Fig. 39 in Seitenansicht wiedergegebenen Ausführungsform der Detektoranordnung 1, bei der der Detektor 12 über einen Knickarm 104 als Positioniervorrichtung 13 durch einen Motor oder manuell positionierbar ist. Die Positioniervorrichtung 13 weist in diesem Fall einen ersten Armabschnitt 102 und einen zweiten Armabschnitt 103 auf, die vorzugsweise gleich lang sind. Der zweite Armabschnitt 103 ist über eine erste Rotationsachse 99, die unterhalb der Auflage 3 liegt, mit einer unterhalb der Auflage 3 angebrachten Befestigung verbunden sowie über eine zweite Rotationsachse 100 mit dem zweiten Armabschnitt 103. Der zweite Armabschnitt 103 ist außerdem mit dem Detektor 12 über die dritte Rotationsachse 101 verbunden, die durch einen Mittelpunkt des Detektors 12 verläuft, wobei der Detektor 12 um die dritte Rotationsachse 101 drehbar ist und in beliebige Winkel zu dem zweiten Armabschnitt 103 positioniert werden kann. Um die erste Rotationsachse 99, die zweite Rotationsachse 100 und die dritte Rotationsachse 101 können jeweils Rotationen um 360° vollführt werden. Durch die Dreiachs-Knickarmkonstruktion kann der Detektor 12 seitlich und unterhalb der Auflage 3 bewegt werden. Der Knickarm 104 kann zusätzlich auch über ein Linearachse, wie im Zusammenhang mit Fig. 38 beschrieben, bewegt werden.

Um den Detektor 12 unterhalb der Auflage 3 und parallel zu dieser zu halten, können der erste Armabschnitt 102 und der zweite Armabschnitt 103 parallel zueinander sowie vertikal zu der Auflage 3 gestellt. Alternativ kann eine Parkstellung 104 angefahren werden, bei der der Detektor 12 parallel zu der Auflage 3 liegt, jedoch der erste Armabschnitt 102 und der zweite Armabschnitt 103 in eine horizontale Position bewegt wurden. Um den Detektor 12 seitlich zu der Auflage 3 zu verfahren, wird der zweite Armabschnitt 103 parallel zu der Auflage 3 gestellt und der zweite Armabschnitt 103 durch die zweite Rotationsachse 100 abgewinkelt.

Fig. 40 zeigt in einer um 90° gegenüber der Ansicht der Fig. 39 gedrehten Ansicht die in Fig. 39 dargestellte Anordnung. Der Knickarm 104 ist an dem mit dem Boden 9 verbundenen Standfuß 10 verbunden, auf dem die Auflage 3 angeordnet ist. Der erste Armabschnitt 102 und der zweite Armabschnitt 103 sind, ebenso wie der Detektor 12, in ihrer Grundposition unterhalb der Auflage 3 angeordnet.

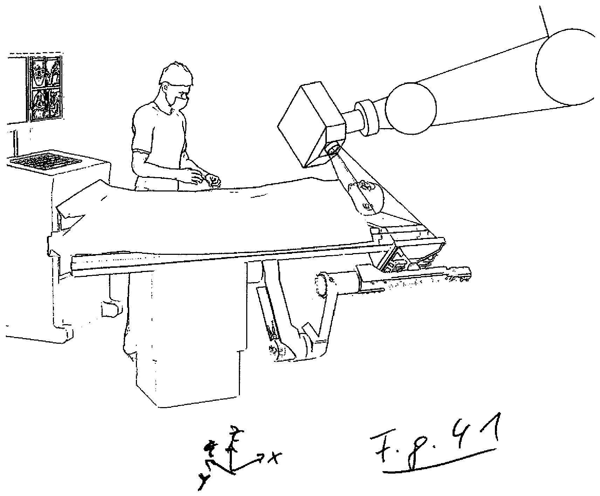

Die Figuren 39 und 40 zeigen sämtliche Merkmale des Patentanspruchs 1. Lediglich zur Erläuterung werden Figuren 41 bis 45 beigefügt, die lediglich der noch besseren Veranschaulichung dienen.

Fig. 41 zeigt hierbei einen stehenden Operateur an einer Säule (einem Standfuß) mit darauf befindlicher Auflage sowie einem Patienten. Ein an der Decke befindliches Röntgengerät (mit Knickarm/Roboterarm) ist auf den Patienten gerichtet, die Röntgenaufnahme wird durch einen Detektor am Ende des zweiten Roboterarms (also an der Positioniervorrichtung 13) vorgenommen.

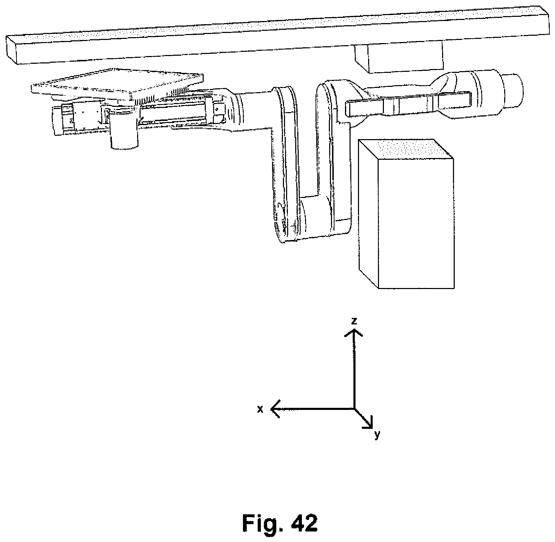

Fig. 42 zeigt eine Explosionsdarstellung eines Standfußes 10 einer Positioniervorrichtung 13 sowie einer Auflage 3. Hierbei ist gut zu sehen, dass die Positioniervorrichtung im Wesentlichen zwischen dem Standfuß 10 sowie der Auflage 3 angeordnet werden kann; somit ist beispielsweise auch eine Nachrüstung der Positioniervorrichtung denkbar.

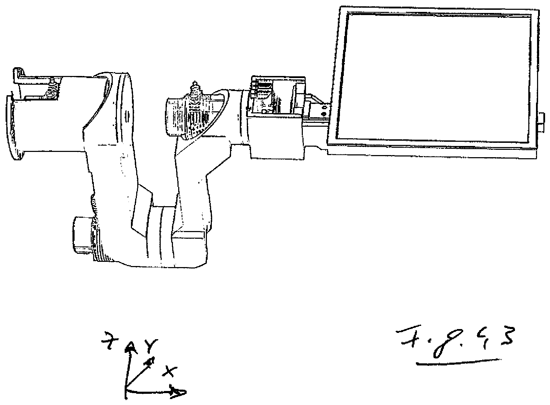

Fig. 43 zeigt eine Normalstellung der Positioniervorrichtung 13 mit am Ende eines Roboterarms/Knickarms befindlichem Detektor 12.

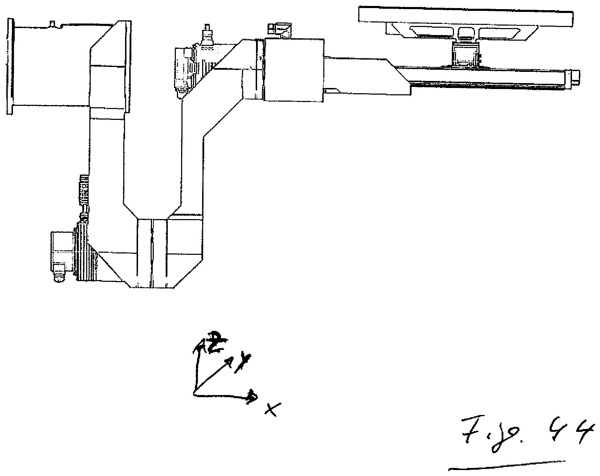

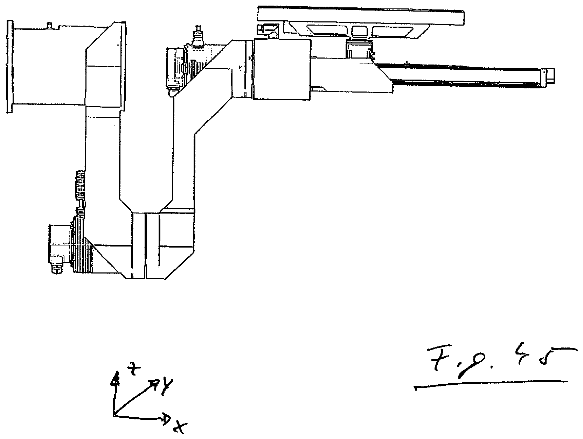

In den Figuren 44 und 45 sind Seitansichten der Positioniervorrichtung gezeigt. Hierbei ist in Fig. 44 ein Linearantrieb am Ende des Roboterarms mittig mit dem Detektor 12 versehen, während in Fig. 45 der Detektor 12 nach links verschoben ist (also in negativer x-Richtung).

Die Figuren 39 bis 45 zeigen also eine Detektoranordnung zum Aufnehmen von Röntgenbildern eines auf einer Auflage eines Tisches befindlichen abzubildenden Objekts, wobei der Tisch zumindest einen verstellbaren Standfuß zum Bewegen der Auflage aufweist, umfassend: einen Detektor zum Erfassen von Röntgenstrahlung und eine Positioniervorrichtung zum Bewegen des Detektors bezüglich des Objekts, wobei der Detektor in mehrere zu dem Objekt raumfeste Aufnahmepositionen bewegbar ist und die Positioniervorrichtung an dem Standfuß derart befestigbar ist, dass die Positioniervorrichtung gemeinsam mit der Auflage bewegt wird und die Positioniervorrichtung einen Knickarm aufweist, der über mindestens eine Rotationsachse und/oder eine Linearachse bewegbar ist.

### Bezugszeichenliste:

- 1: Detektoranordnung
- 2: Röntgenquelle
- 2e: Aufnahmeposition
- 2f: Aufnahmeposition
- 2g: Aufnahmeposition
- 2h: Aufnahmeposition
- 3: Auflage
- 4: Röntgenvorrichtung
- 5: Roboterarm
- 6: erstes Gelenk
- 7: zweites Gelenk
- 8: Decke
- 9: Boden
- 10: Standfuß
- 11: Befestigung
- 12: Detektor
- 12a: Aufnahmeposition
- 12b: Aufnahmeposition
- 12c: Aufnahmeposition
- 12d: Aufnahmeposition
- 12e: Aufnahmeposition
- 12f: Aufnahmeposition
- 12g: Aufnahmeposition
- 12h: Aufnahmeposition
- 13: Positioniervorrichtung
- 14: Röntgenkegelstrahl
- 15: Gehäuse
- 16: drittes Gelenk
- 17: Oberflächennormale des Detektors
- 18: Winkelabweichung
- 19: mittlerer Teil der Detektoranordnung
- 20: Abbildungsfläche
- 20a: Seitenabbildungsfläche
- 21: Seitenelemente
- 22: Bewegungslager
- 23: Schienensystem
- 24: Mittelebene
- 25: weiterer Detektor
- 26: offener Verbindungsbereich
- 27: geschlossener Verbindungsbereich
- 28: Führungsstäbe
- 29: abzubildendes Objekt
- 30: durchstrahltes Material des Seitenelements
- 31: erstes Zielgebiet
- 32: zweites Zielgebiet
- 33: Recheneinheit
- 34: Kabel
- 35: weiteres Kabel
- 36: Ausgabeeinheit
- 37: gerade Schiene
- 38: gebogene Schiene
- 39: Strebe
- 40: weiteres Schienenlager
- 41: weitere gerade Schienen
- 42: Wagen der Röntgenquelle
- 43: Detektorgehäusecontainer
- 44: Räder
- 45: Räder
- 46: Bewegungslager Standfuß/Auflage
- 47: Bewegungslager Auflage/Detektoranordnung
- 48: Detektorcontainer
- 49: Schutzlage
- 50: Zahnstange
- 51: Zahntrieb
- 52: Aufnahmeposition
- 53: Zahnradgleitführung
- 54: Fügestelle
- 55: Seitenwand
- 56: Schlitten
- 57: gebogene Zahnstange
- 58: angetriebene Rotationsachse
- 59: unteres Ende des Seitenteils
- 60: bewegliche Montagefläche
- 61: Führung
- 62: Auslenker-Schlitten
- 63: Verbindung
- 64: Linearantrieb
- 65: seitliche Position
- 66: Drehachse
- 67: Linearantrieb-Schlitten
- 68: Linearführung
- 69: Auslenkerbasis
- 70: abgewinkelte Position des Detektors
- 71: Wagen
- 72: Wagenräder
- 73: Drehachse
- 74: Klapp-/Drehachse
- 75: Roboterarm
- 76: erstes Gelenk
- 77: zweites Gelenk
- 78: drittes Gelenk
- 79: kreisförmige Aufnahmebahn
- 80: elliptische Aufnahmebahn
- 81: Längsachse der Auflage
- 82: Querachse der Auflage
- 83: Drehachse
- 84: durchfahrener Bereich
- 85: Halterung
- 86: Scharnier
- 87: Verbindungsstück
- 88: Schiene im mittleren Teil
- 89: Schiene im Seitenelement
- 90: Rotationsachse
- 91: U-förmige Halterung
- 92: weitere Rotationsachse
- 93: Bodenbefestigung Hubantrieb
- 94: Bewegungsrichtung des Hubantriebs
- 95: Linearbewegung
- 96: Flansch
- 97: Linearachse
- 98: Hubantrieb
- 99: erste Rotationachse
- 100: zweite Rotationsachse
- 101: dritte Rotationachse
- 102: erster Armabschnitt
- 103: zweiter Armabschnitt
- 104: Knickarm in Parkstellung
- 105: Klemmhalterung
- 106: durch den Mittelpunkt des Objekts verlaufende Ebene
- 107: Motor
- 108: zentrale Achse

## Patentansprüche

1. Detektoranordnung (1) zum Aufnehmen von Röntgenbildern eines auf einer Auflage eines Tisches befindlichen abzubildenden Objekts (29), wobei der Tisch zumindest einen verstellbaren Standfuß zum Bewegen der Auflage aufweist, umfassend
einen Detektor (12) zum Erfassen von Röntgenstrahlung,
den Tisch mit dem verstellbaren Standfuß und der Auflage und
eine Positioniervorrichtung (13) zum Bewegen des Detektors (12) bezüglich des Objekts (29), wobei
der Detektor (12) in mehrere zu dem Objekt (29) raumfeste Aufnahmepositionen bewegbar ist und
die Positioniervorrichtung (13) an dem Standfuß derart befestigt ist, dass die Positioniervorrichtung gemeinsam mit der Auflage bewegt wird und
die Positioniervorrichtung (13) einen Knickarm aufweist, **dadurch gekennzeichnet, dass** der Knickarm ein Roboterarm ist, der über mindestens eine Rotationsachse (99, 100, 101) und/oder eine Linearachse bewegbar ist, wobei der Roboterarm mindestens drei Roboterarmachsen (99, 100, 101) aufweist, die parallel zueinander und parallel zu einer Längsrichtung der Auflage angeordnet sind.

2. Detektoranordnung nach Anspruch 1, **dadurch gekennzeichnet, dass** die Positioniervorrichtung (13) zwischen dem Standfuß (10) und der Auflage (3) befestigt ist.

3. Detektoranordnung nach einem der vorhergehenden Ansprüche, **dadurch gekennzeichnet, dass** die Auflage (3) zumindest bereichsweise aus einem röntgendurchlässigen Material besteht.

4. Detektoranordnung nach einem der vorhergehenden Ansprüche, **dadurch gekennzeichnet, dass** der Standfuß (10) so ausgestaltet ist, dass Auflage (3) und Positioniervorrichtung (13) gemeinsam in der Höhe und/oder in der Neigung verstellbar sind.

5. Detektoranordnung nach Anspruch 4, **dadurch gekennzeichnet, dass** die Neigung in einer oder zwei Raumrichtungen (x und/oder y) verstellbar ist.

6. Detektoranordnung nach einem der vorhergehenden Ansprüche, **dadurch gekennzeichnet, dass** die Auflage (3) auf genau einem Standfuß (10) gehalten ist.

7. Detektoranordnung nach einem der vorhergehenden Ansprüche, **dadurch gekennzeichnet, dass** Auflage (3) und Positioniervorrichtung (13) derart bezüglich des Standfußes (10) positioniert sind, dass ein Operateur seine Beine unterhalb der Auflage (3) unterbringen kann.

8. Detektoranordnung nach einem der vorhergehenden Ansprüche, **dadurch gekennzeichnet, dass** die Auflage (3) eine Längsrichtung (x) aufweist und die Auflage (3) bezüglich des Standfußes (10) in dieser Längsrichtung (x) entweder zusammen mit der Positioniervorrichtung (13) oder unabhängig von der Positioniervorrichtung (13) bewegbar ist.

9. Detektoranordnung nach Anspruch 8, **dadurch gekennzeichnet, dass** der Detektor (12) mittels der Positioniervorrichtung (13) zusätzlich translatorisch (vorzugsweise in x- und/oder y-Richtung) bezüglich der Auflage (3) positionierbar ist.

10. Detektoranordnung nach Anspruch 9, **dadurch gekennzeichnet, dass** die translatorische Positionierbarkeit in mindestens einer Raumrichtung (x) gegeben ist.

11. Detektoranordnung nach Anspruch 10, **dadurch gekennzeichnet, dass** diese Raumrichtung eine Längsrichtung (x) der Auflage (3) und damit parallel zu den drei Roboterarmachsen (99, 100, 101) ist.

12. Detektoranordnung nach Anspruch 10, **dadurch gekennzeichnet, dass** die translatorische Positionierbarkeit in zwei oder drei Raumrichtungen gegeben ist.

13. Detektoranordnung nach einem der vorhergehenden Ansprüche, **dadurch gekennzeichnet, dass** der Detektor (12) im Wesentlichen flächenförmig ausgestaltet ist und in einer Ausgangslage parallel unterhalb der Auflage (3) so positionierbar ist, dass die Beine eines Operateurs zwischen Fußboden und Detektor (12) angeordnet werden können.

14. Detektoranordnung nach einem der vorhergehenden Ansprüche, **dadurch gekennzeichnet, dass** die Positioniervorrichtung derart ausgestaltet ist, dass der Detektor (12) von mindestens zwei Seiten der Auflage (3) aus von unterhalb der Auflage (3) nach oberhalb der Auflage (3) bewegbar ist.

15. Detektoranordnung (1) nach einem der vorhergehenden Ansprüche, **dadurch gekennzeichnet, dass** der Detektor (12) durch einen Linearantrieb und/oder durch einen Motor auf der Positioniervorrichtung (13) verfahrbar ist, wobei vorzugsweise der Detektor (12) durch eine den Motor steuernde Recheneinheit (33) automatisiert verfahrbar ist.

## Claims

1. A detector arrangement (1) for recording X-ray pictures of an object (29) which is located on a rest of a table and which is to be imaged, wherein the table comprises at least one adjustable support foot for moving the rest, comprising
a detector (12) for detecting X-ray radiation,
the table with the adjustable support foot and the rest, and
a positioning device (13) for moving the detector (12) with respect to the object (29), wherein the detector (12) is movable into several recording positions which are spatially fixed with respect to the object (29) and
the positioning device is fastened on the support foot in a manner such that the positioning device is moved together with the rest and
the positioning device comprises an articulated arm, **characterized in that** the articulated arm is a robot arm which is movable via at least one rotation axis (99, 100, 101) and/or a linear axis, wherein the robot arm comprises at least three robot arm axes (99, 100, 101), which are arranged parallel to each other and parallel to a longitudinal direction of the rest.

2. A detector arrangement according to claim 1, **characterised in that** the positioning device (13) is fastened between the support foot (10) and the rest (3).

3. A detector arrangement according to one of the preceding claims, **characterised in that** the rest (3) consists at least in regions of a material transparent to X-rays.

4. A detector arrangement according to one of the preceding claims, **characterised in that** the support foot (10) is designed such that the rest (3) and the positioning device (13) together are adjustable in height and/or in inclination.

5. A detector arrangement according to claim 4, **characterised in that** the inclination is adjustable in one or two spatial directions (x and/or y).

6. A detector arrangement according to one of the preceding claims, **characterised in that** the rest (3) is held on exactly one support foot (10).

7. A detector arrangement according to one of the preceding claims, **characterised in that** the rest (3) and the positioning device (13) are positioned with respect to the support foot (10) in a manner such that an operator can accommodate his legs below the rest (3).

8. A detector arrangement according to one of the preceding claims, **characterised in that** the rest (3) has a longitudinal direction (x), and the rest (3) with respect to the support foot (10) is movable in this longitudinal direction (x) either together with the positioning device (13) or independently of the positioning device (13).

9. A detector arrangement according to claim 8, **characterised in that** the detector (12) by way of a positioning device (13) can be positioned additionally translatorily (preferably in the x-direction and/or y-direction) with respect to the rest (3).

10. A detector arrangement according to claim 9, **characterised in that** the translatory positionablity is given in at least one spatial direction (x).

11. A detector arrangement according to claim 10, **characterised in that** this spatial direction is a longitudinal direction (x) of the rest (3) and, thus, is parallel to the three robot arm axes (99, 100, 101).

12. A detector arrangement according to claim 10, **characterised in that** the translatory positionability is given in two or three spatial directions.

13. A detector arrangement according to one of the preceding claims, **characterised in that** the detector (12) is designed in an essentially two-dimensional manner and in an initial position can be positioned parallel below the rest (3) such that the legs of an operator can be arranged between the floor and the detector (12).

14. A detector arrangement according to one of the preceding claims, **characterised in that** the positioning device is designed in a manner such that the detector (12) is movable from at least two sides of the rest (3) from below the rest (3) to above the rest (3).

15. A detector arrangement according to one of the preceding claims, **characterised in that** the detector (12) is movable on the positioning device (13) by way of a linear drive and/or by way of a motor, wherein preferably the detector (12) is movable in an automated manner by way of a computation unit (33) controlling the motor.

## Revendications

1. Agencement de détecteurs (1) pour la prise d'images radiographiques d'un objet (29) à représenter, se trouvant sur un support d'une table, où la table présente au moins un pied réglable pour le déplacement du support, comprenant un détecteur (12) pour la détection du rayonnement radiographique,
la table avec le pied réglable et le support et
un dispositif de positionnement (13) pour le déplacement du détecteur (12) par rapport à l'objet (29), où
le détecteur (12) peut être déplacé dans plusieurs positions de prises fixes dans l'espace par rapport à l'objet (29), et
le dispositif de positionnement (13) est fixé au pied de telle manière que le dispositif de positionnement est déplacé conjointement avec le support, et
le dispositif de positionnement (13) présente un bras articulé, **caractérisé en ce que** le bras articulé est un bras de robot,
qui peut être déplacé selon au moins un axe de rotation (99, 100, 101) et/ou un axe linéaire, où le bras de robot présente au moins trois axes de bras de robot (99, 100, 101) qui sont disposés parallèlement les uns par rapport aux autres et sont parallèles à une direction longitudinale du support.

2. Agencement de détecteurs selon la revendication 1, **caractérisé en ce que** le dispositif de positionnement (13) est fixé entre le pied (10) et le support (3).

3. Agencement de détecteurs selon l'une des revendications précédentes, **caractérisé en ce que** le support (3) est constitué au moins par endroits d'un matériau perméable aux rayons X.

4. Agencement de détecteurs selon l'une des revendications précédentes, **caractérisé en ce que** le pied (10) est conçu de telle manière que le support (3) et le dispositif de positionnement (13) sont réglables conjointement en hauteur et/ou en inclinaison.

5. Agencement de détecteurs selon la revendication 4, **caractérisé en ce que** l'inclinaison est réglable dans une ou deux directions dans l'espace (x et/ou y).

6. Agencement de détecteurs selon l'une des revendications précédentes, **caractérisé en ce que** le support (3) est maintenu en place sur exactement un pied (10).

7. Agencement de détecteurs selon l'une des revendications précédentes, **caractérisé en ce que** le support (3) et le dispositif de positionnement (13) sont positionnés par rapport au pied (10) de telle manière qu'un opérateur peut mettre ses jambes en dessous du support (3).

8. Agencement de détecteurs selon l'une des revendications précédentes, **caractérisé en ce que** le support (3) présente une direction longitudinale (x) et le support (3) peut se déplacer par rapport au pied (10) dans cette direction longitudinale (x) soit conjointement avec le dispositif de positionnement (13), soit de manière indépendante par rapport au dispositif de positionnement (13).

9. Agencement de détecteurs selon la revendication 8, **caractérisé en ce que** le détecteur (12) peut être positionné au moyen du dispositif de positionnement (13) en outre de manière translatoire (de préférence dans la direction x et/ou la direction y) par rapport au support (3).

10. Agencement de détecteurs selon la revendication 9, **caractérisé en ce que** le positionnement de manière translatoire est donné dans au moins une direction dans l'espace (x).

11. Agencement de détecteurs selon la revendication 10, **caractérisé en ce que** cette direction dans l'espace est une direction longitudinale (x) du support (3) et est par conséquent parallèle aux trois axes de bras de robot (99, 100, 101).

12. Agencement de détecteurs selon la revendication 10, **caractérisé en ce que** le positionnement de manière translatoire est donné dans deux ou trois directions dans l'espace.

13. Agencement de détecteurs selon l'une des revendications précédentes, **caractérisé en ce que** le détecteur (12) est conçu dans l'ensemble de forme plane et peut être positionné dans une position de départ parallèle en dessous du support (3) de telle manière que les jambes d'un opérateur peuvent être disposées entre le sol et le détecteur (12).

14. Agencement de détecteurs selon l'une des revendications précédentes, **caractérisé en ce que** le dispositif de positionnement est conçu de telle manière que le détecteur (12) peut être déplacé à partir d'au moins deux côtés du support (3) depuis du dessous du support (3) jusque au-dessus du support (3).

15. Agencement de détecteurs (1) selon l'une des revendications précédentes, **caractérisé en ce que** le détecteur (12) peut être déplacé par un entraînement linéaire et/ou par un moteur sur le dispositif de positionnement (13), où de préférence, le détecteur (12) peut être déplacé de manière automatisée par une unité de calcul (33) commandant le moteur.
